# EUROPEAN PATENT SPECIFICATION

(11) **EP 3 352 739 B1**
(45) Date of publication and mention of the grant of the patent: **27.10.2021**
(21) Application number: 16849647.9
(22) Date of filing: 23.09.2016
(51) Int. Cl.: A61K 9/08, A61K 9/107, A61K 47/10, A61K 31/385, A61P 27/02, A61K 47/18, A61P 27/10, A61P 27/12

(54) **LIPOIC ACID CHOLINE ESTER COMPOSITIONS AND METHODS TO GENERATE BIOCOMPATIBLE OPHTHALMIC FORMULATIONS**
LIPONSÄURE-CHOLIN-ESTER-ZUSAMMENSETZUNGEN UND VERFAHREN ZUR ERZEUGUNG BIOKOMPATIBLER AUGENFORMULIERUNGEN
COMPOSITIONS D'ESTER DE CHOLINE D'ACIDE LIPOÏQUE ET PROCÉDÉS POUR GÉNÉRER DES FORMULATIONS OPHTALMIQUES BIOCOMPATIBLES

(30) Priority: 24.09.2015 US 201562222844 P
(43) Date of publication of application: 01.08.2018
(73) Proprietor: Novartis AG, 4056 Basel (CH)
(72) Inventor: BARMAN, Shikha P., Bedford, Massachusetts 01730 (US); WARD, Kevin L., Arlington, Massachusetts 02476 (US); CRAWFORD, Kathryn S., Andover, Massachusetts 01810 (US); BURNS, William R., North Richland Hills, Texas 76180 (US)
(74) Representative: Stierwald, Michael
(86) International application number: PCT/US2016/053225
(87) International publication number: WO 2017/053646

(56) References cited:
- WO-A1-2015/134510

## Description

### I. FIELD OF THE INVENTION

The present invention generally relates to ophthalmic lipoic acid choline ester compositions and processes to produce biocompatible, stable and non-irritating eye-drop formulations. The compositions herein are contemplated as therapies for (but not limited to) ocular disorders such as presbyopia, dry eye, cataracts, and age-related macular degeneration.

### II. BACKGROUND OF THE INVENTION

Lipoic acid choline ester (LACE) is a chemically synthesized derivative of R-α-Lipoic Acid.

Lipoic acid, also known as thioctic acid, is an eight carbon fatty acid with a disulfide linkage joining the carbons 6 and 8 to form an 1, 2-dithiolane ring. The acid forms optical isomers of which the isomer R-α-lipoic acid is the most biologically active.

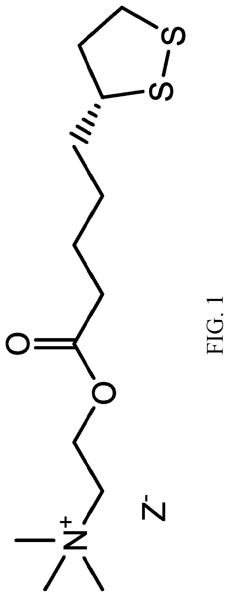

Lipoic Acid Choline Ester (LACE, chemical structure, see FIG. 1) was designed to permeate biological membranes with the incorporation of the cationic choline head group. While lipoic acid does not permeate the cornea, the choline ester derivative of lipoic acid permeates the cornea, is hydrolyzed by corneal esterases and is transformed into the biologically active lipoic acid. LACE has been formulated into an ophthalmic solution to be applied twice daily as an eye-drop to treat presbyopia.

LACE, which is a prodrug consisting of lipoic acid and choline, is a unique molecule to treat presbyopia. Lipoic acid (LA) is the active ingredient and the choline head group serves to aid permeability into the eye. The bonds between LA and choline are hydrolyzed by esterases in the tear film and cornea after the eye drop is administered. The free lipoic acid enters the eye and ultimately reaches the lens. There it is reduced to dihydrolipoic acid by endogenous oxidoreductases which then cause hydrolysis of the cytosolic proteins within the superficial elongated lenticular cells. This protein cleavage allows a free flow of cytosol and reversal of the oxidative processes associated with the age-related stiffening of the lens. It is expected that ophthalmic solutions prepared from LACE will enable accommodation and improve near vision focus in persons with presbyopia, the age-related loss of accommodation.

Presbyopia is an age-related inability to focus on near objects; this condition is caused by physiological changes in the microstructure of the lens resulting in loss of flexibility in the auto-adjustment of focal length and curvature of the lens to bring the visual object under focus. This condition is corrected by corrective lenses. It has been reported that lipoic acid choline ester ("LACE") (see e.g., U.S. Patent No. 8,410,462) can restore near vision.

Supporting this claim are *ex-vivo* studies that demonstrated that lens softening can be induced pharmacologically in human donor lenses using the protein disulfide reducing agent dithiothreitol (DTT), and in mouse lenses with lipoic acid.

This mechanism of action allows the contemplation of treatment of multiple ocular diseases and disorders. These disorders are, but not limited to, presbyopia, age-related macular degeneration, cataract and dry eye.

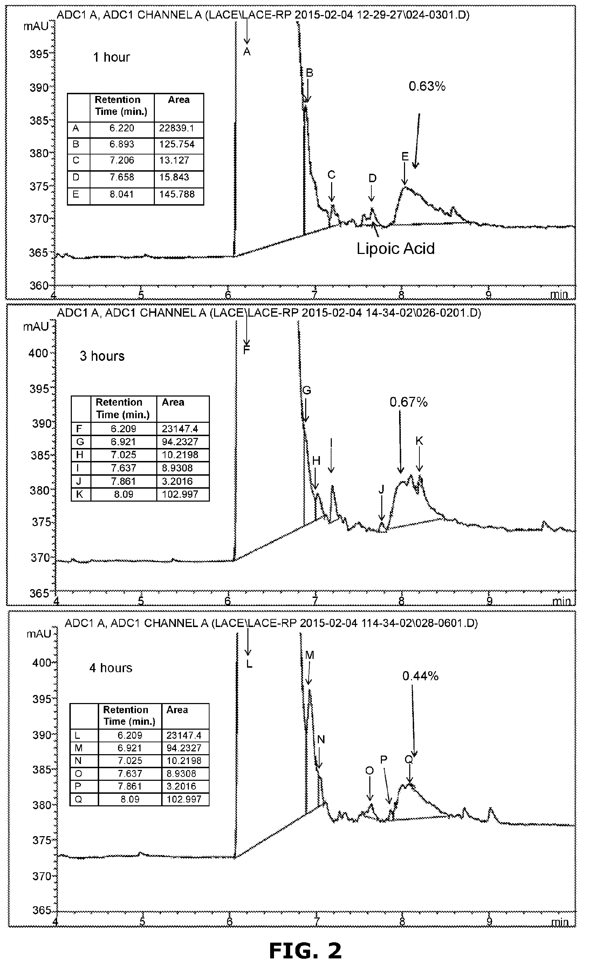

An issue that has rendered formulation of LACE problematic has been the propensity to destabilize by ring-opening of the dithiolane linkage to form oxidized species that compromise the activity of the molecule. At room temperature, LACE rapidly degrades into oxidized species (see "HPLC Chromatogram of LACE Ophthalmic Solution with Degradation Products", see FIG. 2). Even when stored at refrigerated temperatures, rapid oxidation occurs in storage as early as 1 week, compromising the utility of the molecule as a drug product. For LACE Ophthalmic Solution (also referred to as EV06 Ophthalmic Solution) to be utilized in its fullest potential as a drug product, it was critical that the aqueous formulation be stabilized in storage and during use.

Another issue that confounded the pharmaceutical development of LACE ophthalmic solutions was incidences of ocular surface irritation observed *in-vivo* in a rabbit irritation model. The invention details unexpected parameters that contributed to, or caused ocular irritation and processes to eliminate or minimize these parameters. These parameters were not related to the formulation composition or properties of the drug substance, factors that normally correlated or attributed to ocular irritation.

The compositions and methods described within describe methods to stabilize ophthalmic LACE formulations long-term. Also described are unanticipated discoveries as to the cause of irritation of LACE formulations formulated under certain process conditions. Also unobvious were critical process parameters identified as key factors in the generation of final, comfortable ophthalmic solutions of LACE (EV06 Ophthalmic Solution).

The final process conditions minimized the formation of the degradation species and minimized the formation of species that were attributed to ocular irritation.

### III. DESCRIPTION OF THE FIGURES

FIG. 1: Chemical Structure of Lipoic Acid Choline Ester.
FIG. 2: LACE micellar species at 8.1 minutes at 1, 3 and 4 hours of mixing, formulation KW-LACE-01-86-2.
FIG. 3: LACE micellar species at 8.1 minutes at 6, 8 and 24 hours of mixing, formulation KW-LACE-01-86-2.
FIG. 4: Micellar LACE species (peak denoted by arrow) are highest when mixed at refrigerated temperatures
FIG. 5A: High Micellar LACE Concentrations (denoted by large peak between 7.9-8.5 minutes on HPLC) is correlated to "clumped" LACE chloride API.
FIG. 5B: Lower micellar LACE (peak denoted by arrow) is correlated with non-clumped LACE API.
FIG. 6: Effect of Alanine and pH.
FIG. 7 is a plot of formulations comparing the following variables: (a) Control (original) versus Control + 0.25% sodium chloride, Control + 0.25% sodium chloride, w/o glycerin, (b) Control (original) versus control, w/o benzalkonium chloride (BAC), (c) Control (original) versus original formulation without glycerin.
FIG. 8 shows the effect of sulfite on LACE stability at 57°C. Sulfite-containing formulations were prepared at concentrations 0.05% sulfite (■, ▲) and 0.1% sulfite (x) at pH 4 and 4.5. Addition of sulfite did not stabilize the original formulation (◆).
FIG. 9 further explores the potentially stabilizing effect of eliminating benzalkonium chloride. All formulation variations without benzalkonium chloride were superior to the control original formulation (pH 4.5). Formulation variations were BAC-free compositions at pHs 4, 4.5 (▲,■), no glycerin/no BAC + 0.9% sodium chloride (x), no BAC + 0.05% sulfite at pHs 4 and 4.5 (●, ■).
FIG 10: Effect of Eliminating Glycerin on LACE Stability.
FIG. 11: Effect of Buffered Compositions on LACE Stability.
FIG. 12A: Correlation of Irritation Score (in a rabbit irritation model) with % LACE Micellar Species Measured by HPLC-UV.
FIG. 12B: Correlation of Irritation Score (in a rabbit irritation model) with % LACE Micellar Species Measured by HPLC-ELSD.

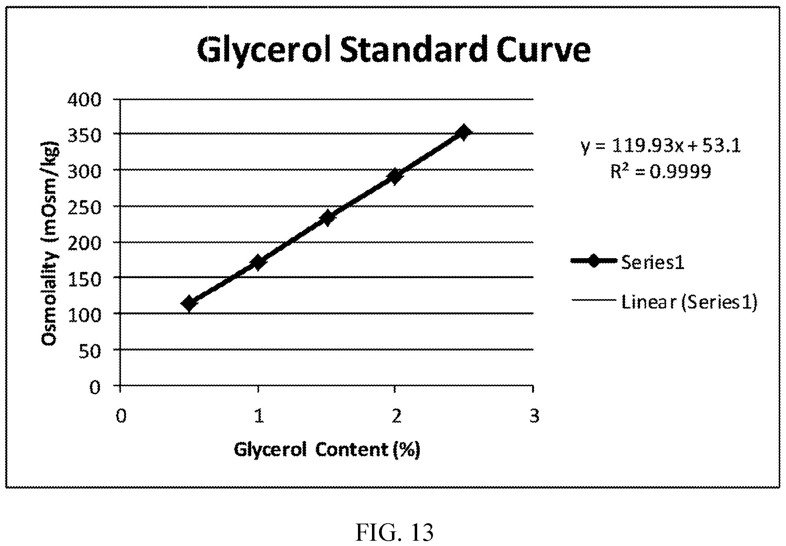

FIG.13: Glycerol Osmolality Standard Curve.

### IV. BRIEF SUMMARY OF THE INVENTION

The present invention achieves two primary objectives: (a) to generate ophthalmic solutions of LACE that are stable for at least a year at refrigerated storage temperatures of 2-5°C, and (b) to generate and optimize process parameters to prepare ophthalmic solutions of LACE, that are non-irritating to the eye.

The chemical structure of LACE demonstrates two points of degradation. One is ring opening of the diothiolane ring and the other is oxidative and hydrolytic degradation. As mentioned earlier, LACE interacts with oxygen to rapidly generate oxidized species. In water, LACE is also susceptible to hydrolysis of the ester linkage to generate Lipoic Acid and Choline. The rate at which hydrolysis occurs is correlated to temperature; hydrolysis is less at lower temperatures and pH.

Studies were performed on LACE ophthalmic solution derivatives, also called EV06 Ophthalmic Solution, stored in permeable LDPE (low density polyethylene) eye-dropper bottles, which are gas permeable. Described herein are methods that the inventors have developed to minimize oxidation of the compounded LACE solution during storage.

Additionally, extensive compatibility studies of excipient mixtures with LACE established the criticality of certain excipients as stabilizing factors, the role of pH in stabilization of the hydrolysis of LACE in water, as well as the effect of osmolality-adjusting agents such as sodium chloride and glycerol. Most importantly, the stabilizing effect of Alanine to LACE, as opposed to citrate, phosphate and borate has been described in the present invention.

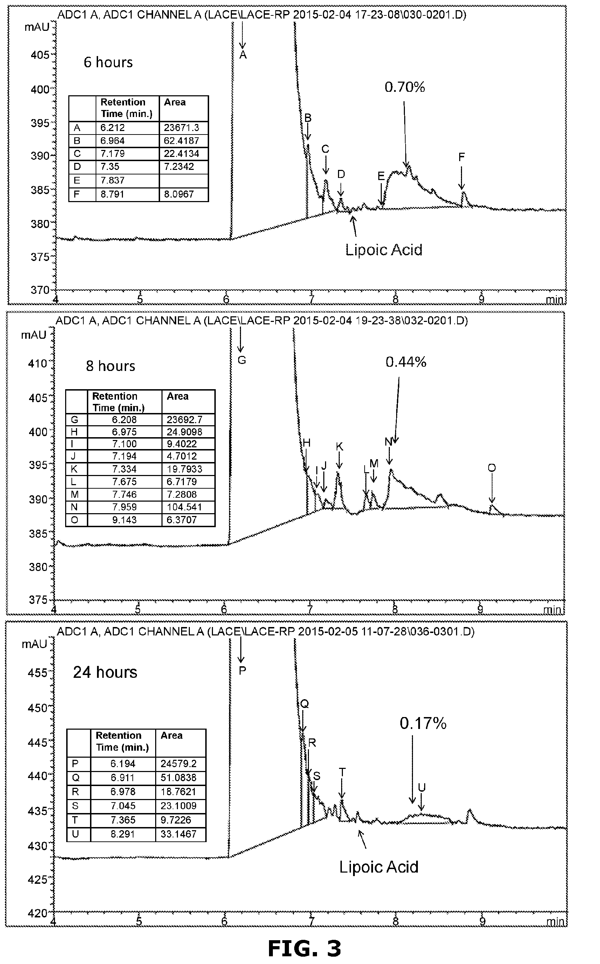

While searching for causes for irritation, it was discovered that LACE, when dissolved in water, forms micelles and micellar aggregates, common to compounds that are amphiphilic in nature. As definition, examples of micelle-forming compounds are phosphatidyl choline, pegylated phosphatidyl choline, PEG-stearate, sorbitol, etc. While the micelle-formation phenomenon of LACE is not unexpected due to the amphiphilic nature of the molecule, the formation of these aggregates at lower temperatures were surprising. The presence of the aggregates was measured by a RP-HPLC method developed in-house. The measurement could be performed both with HPLC-UV (FIG. 3A) and HPLC-ELSD (FIG. 3B)

LACE aqueous solutions formed gel-like structures at refrigerated temperatures (2-5°C). It is also expected that the number and aggregation of these micellar assemblies increase with increase in concentration of the micelle-forming drug. The inventors have correlated the extent of micellar aggregation of LACE with ocular surface irritation, a result that was unanticipated and surprising, since micellar vehicles are often contemplated as drug delivery systems for insoluble compounds. Thus, this is the first reported account of irritation correlated to micellar aggregates. Once discovered, this phenomenon needed to be minimized through compounding methods to correlate with comfort.

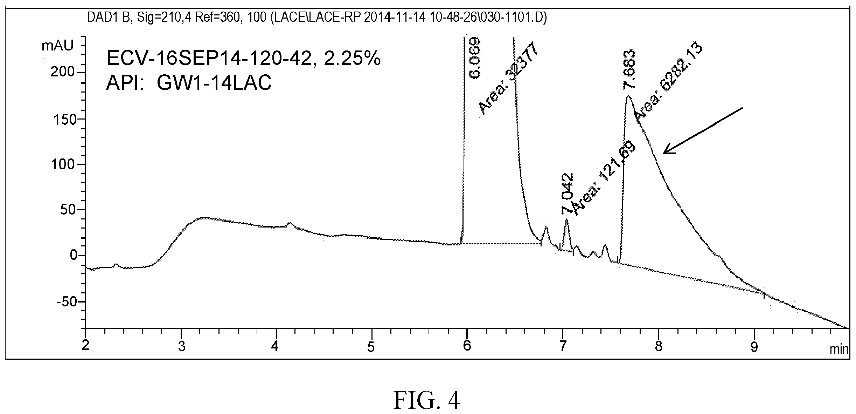

The formation of micellar aggregates appeared to be correlated to the temperature of compounding (FIG. 4). The formation of self-assemblies is a thermodynamic phenomenon, correlated to efficient lowering of surface free energy to achieve a minimized energy state. When LACE was compounded in water at a lower temperature (5°C), aggregates that had a gel-like consistency were formed. Compositions formulated at refrigerated temperatures were extremely irritating to the eye. The aggregated state could be quantitated by a RP-HPLC method (see chromatogram shown in FIG. 3A-3B). A series of investigative experiments demonstrated no presence of polymers or oligomers, when measured by extensive Size Exclusion Chromatography (SEC). Other investigations tested ocular irritation as a function of processes conducted in the presence of ambient air or in the presence of nitrogen. There was no correlation of irritation to air or nitrogen. Both were equally comfortable when formulated at room temperature, although the degradation products were higher in the presence of air. When LACE was compounded at room temperature, the micellar aggregation was lower as quantitated by the HPLC method. LACE compounded at room temperature generated solutions that were comfortable and non-irritating.

Also unanticipated were the "disentangling" of the micellar aggregates. The aggregates formed in LACE aqueous compositions could be "disentangled" as the solutions were left to equilibrate on the benchtop at room temperature, as measured by HPLC. Additional experiments showed that the vigorous mixing achieved de-aggregation. Thus, it was proved that these species were not permanent species with covalent linkages, but rather a self-assembly of LACE aggregates that appeared to have a lower concentration at room temperature, compared to 5°C. LACE aqueous solutions when frozen, formed a stringy consistency. These solutions, when brought up to room temperature and stored at this temperature looked like homogeneous solutions again, lending further credence to concept of temperature dependence of self-assembly.

However, once compounded, aggregate-free solutions of LACE could be stored in refrigerated conditions to minimize oxidative and hydrolytic degradation. It was established through stability studies that the ideal storage temperature of LACE is 2-5°C, to minimize degradation events.

The ideal compounding conditions were determined to be at room temperature (22-25°C) to yield the least irritating solution and the ideal storage condition was determined to be between 2-5°C, to achieve a stable, comfortable ophthalmic solution of LACE for presbyopia.

To further aid in the stabilization of ophthalmic solutions prepared from LACE, oxygen scavenger packets were placed in mylar, impermeable pouches with the LDPE ophthalmic bottles to prevent oxidation-induced degradation. Extensive stability studies demonstrated achievement of a year's stability of EV06 Ophthalmic Solutions.

Also described in this present invention are embodiments of various compositions that stabilize LACE, including other types of aqueous preparations including liposomes, emulsions compounded for the primary purpose of stabilization of the drug.

### V. DETAILED DESCRIPTION OF THE INVENTION

### A. DEFINITIONS OF TERMS

The term "EV06," "LACE" or "lipoic acid choline ester" is understood to have the chemical structure as shown in FIG. 1.

As used herein, LACE formulations refer to lipoic acid choline ester formulations. For example, LACE 1.5% formulation refers to a formulation having 1.5% lipoic acid choline ester by weight of the formulation. Alternatively, EV06 Ophthalmic Solution, 1.5% refers to a formulation that is comprised of 1.5% lipoic acid choline ester.

As used herein, a "derivative" of lipoic acid choline ester is understood as any compound or a mixture of compounds, excluding lipoic acid and choline, formed from reacting lipoic acid choline ester with a non-aqueous pharmaceutical excipient.

As used herein, the term "self-assembly" denotes a thermodynamic assembling of molecules to achieve the most stable energy state. An example of self-assembly are micelles formed in water, typically formed by molecules with a hydrophobic component and a hydrophilic component. The hydrophilic component of the molecule is on the surface of micelles, while the interior contains the hydrophobic parts; for LACE, the choline head group is on the surface of the micelle.

Unless specifically stated or obvious from context, as used herein, the term "excipient" refers to pharmaceutically acceptable excipient.

The term "treating" refers to administering a therapy in an amount, manner, or mode effective to improve a condition, symptom, or parameter associated with a disease or disorder.

The term "preventing" refers to precluding a patient from getting a disorder, causing a patient to remain free of a disorder for a longer period of time, or halting the progression of a disorder, to either a statistically significant degree or to a degree detectable to one skilled in the art.

The term "therapeutically effective amount" refers to that amount of an active ingredient (e.g., LACE or derivatives thereof), which results in prevention or delay of onset or amelioration of symptoms of an ocular disease or disorder (e.g., presbyopia) in a subject or an attainment of a desired biological outcome, such as improved accommodative amplitude or another suitable parameter indicating disease state.

As used herein, the term "shelf-stability" or "shelf stable" is understood as a character of or to characterize a composition or an active ingredient (e.g., LACE or derivatives thereof) that is substantially unchanged upon storage. Methods for determining such shelf-stability are known, for example, shelf-stability can be measured by HPLC to determine the percentage of the composition or active ingredient (e.g., lipoic acid choline ester) that remains or has been degraded in a formulation following storing the formulation for a certain period of time. For example, shelf stable pharmaceutical composition can refer to a composition, which after being stored as per pharmaceutical standard (ICH) has at least 90% (e.g., 91%, 92%, 93%, 94%, 95%, 96%, 97%, 98%, 99%, or greater than 99%) of the active ingredient (e.g., lipoic acid choline ester) present in the composition as measured by HPLC.

As used herein, the term "relative retention time" or "RRT" of a compound can be calculated using the equation "RRT = (t₂ - t₀) / (t₁ - t₀)," wherein t₀ = void time, t₁ = retention time of lipoic acid choline ester, and t₂ = retention time of the compound, as measured by HPLC.

The term "subject" as used herein generally refers to an animal (e.g., a pet) or human, including healthy human or a patient with certain diseases or disorders (e.g., presbyopia).

### VI. LACE COMPOSITIONS AND EMBODIMENTS

As described herein, the proposed invention provides embodiments of pharmaceutical compositions comprising therapeutically effective amounts of lipoic acid choline ester, excipients, buffers and conditions that are compatible and methods and processes that result in biocompatible (non-irritating) and stable solutions suitable as ophthalmic eye-drops.

Concentration of lipoic acid choline ester or derivatives thereof in the pharmaceutical composition can be any concentration from 0.01-0.1%, 0.1% to 10% (e.g., 0.1%, 1%, 2%, 3%, 4%, 5%, 6%, 7%, 8%, 9%, 10%, or any ranges based on these specified numeric values) by weight of the composition. In some embodiments, the concentration of the lipoic acid choline ester in the pharmaceutical composition is 1%. In some embodiments, the concentration of the lipoic acid choline ester in the pharmaceutical composition is 3%. In some embodiments, the concentration of the lipoic acid choline ester in the pharmaceutical composition is 4%. The preferred range of LACE in the composition is 1-3%. Within this range, the preferred composition range is 1.5-4%.

In another embodiment, the effective compositions in the proposed invention are aqueous formulations contain LACE and Alanine, with Alanine at concentrations between 0.1-0.5%, 0.5%-1%, 1%-1.5%, 1.5%-3%. Within this range, the preferred composition is 0.5% Alanine and 1.5% LACE.

In a preferred embodiment, the effective LACE and Alanine-containing composition contains benzalkonium chloride as a preservative at concentrations between 30-150 ppm.

In another embodiment, other preservatives such as polyquartenium, SofZia is included in the LACE aqueous formulation as preservatives at concentrations approved for human use by the FDA. Other preservatives can be 2-phenyl ethanol, boric acid, disodium edetate.

In another embodiment, the LACE-containing ophthalmic solution is a single dose sterile product with no preservative.

Since self-assembled micellar solutions of LACE dissolved in water at high concentrations may demonstrate some irritation, a method to render biocompatible solutions may be encapsulation in liposomes. In this case, LACE will be contained in the interior of the liposomes. Liposomes are generally biocompatible with the ocular surface.

In another embodiment, the pharmaceutical composition has glycerol in concentrations of 0.1%-10%. In a preferred embodiment, the composition has a glycerol concentration of 0.1-5%.

In some embodiments, the preservative is benzalkonium chloride and the biochemical energy source is alanine. In some embodiments, the lipoic acid choline ester has a counter ion selected from the group consisting of chloride, bromide, iodide, sulfate, methanesulfonate, nitrate, maleate, acetate, citrate, fumarate, hydrogen fumarate, tartrate (e.g., (+)-tartrate, (-)-tartrate, or a mixture thereof), bitartrate, succinate, benzoate, and anions of an amino acid such as glutamic acid.

Suitable buffer agent can be any of those known in the art that can achieve a desired pH (e.g., described herein) for the pharmaceutical composition. Non-limiting examples include phosphate buffers (e.g., sodium phosphate monobasic monohydrate, sodium phosphate dibasic anhydrous), acetate buffer, citrate buffer, borate buffers, and HBSS (Hank's Balanced Salt Solution). Suitable amount of a buffer agent can be readily calculated based on a desired pH. In any of the embodiments described herein, the buffer agent is in an amount that is acceptable as an ophthalmic product. However, in some embodiments, the pharmaceutical composition does not include a buffer agent. In some embodiments, the pH of the aqueous solution or the final pharmaceutical composition is adjusted with an acid (e.g., hydrochloride acid) or a base (e.g., sodium hydroxide) to the desired pH range (e.g., as described herein).

In other embodiments, the buffer system could be selected from borate buffers, phosphate buffers, calcium buffers and combinations and mixtures thereof. In the preferred embodiment, the buffer is an amino acid buffer. In another preferred embodiment, the amino acid buffer is comprised of Alanine.

In some embodiments, the lipoic acid choline ester has a counter ion selected from the group consisting of chloride, bromide, iodide, sulfate, methanesulfonate, nitrate, maleate, acetate, citrate, fumarate, hydrogen fumarate, tartrate (e.g., (+)-tartrate, (-)-tartrate, or a mixture thereof), succinate, benzoate, and anions of an amino acid such as glutamic acid. Other counter ions are stearate, propionate and furoate.

In some embodiments, the ophthalmic formulation has a pH of 4 to 8. In some embodiments, the ophthalmic formulation has a pH of 4.5. In some embodiments, the ophthalmic formulation comprises at least one ingredient selected from the group consisting of a biochemically acceptable energy source, a preservative, a buffer agent, a tonicity agent, a surfactant, a viscosity modifying agent, and an antioxidant.

In some embodiments, the pharmaceutical composition contains an anti-oxidant. In some preferred embodiments, the anti-oxidant is comprised of ascorbate. In another preferred embodiment, the anti-oxidant contains glutathione. Suitable antioxidant can be any of those known in the art. Non-limiting examples include ascorbic acid, L-ascorbic acid stearate, alphathioglycerin, ethylenediaminetetraacetic acid, erythorbic acid, cysteine hydrochloride, N-acetylcysteine, L-carnitine, citric acid, tocopherol acetate, potassium dichloroisocyanurate, dibutylhydroxytoluene, 2,6-di-t-butyl-4-methylphenol, soybean lecithin, sodium thioglycollate, sodium thiomalate, natural vitamin E, tocopherol, ascorbyl pasthyminate, sodium pyrosulfite, butylhydroxyanisole, 1,3-butylene glycol, pentaerythtyl tetrakis[3-(3,5-di-t-butyl-4-hydroxyphenyl)]propionate, propyl gallate, 2-mercaptobenzimidazole and oxyquinoline sulfate. Suitable amount of antioxidant can be in the range of 0.1% to 5% (e.g., 0.1%, 0.5%, 1%, 2%, 3%, 4%, 5%, or any ranges based on these specified numeric values) by weight of the composition. In any of the embodiments described herein, the antioxidant is in an amount that is ophthalmically acceptable.

In some embodiments, the pharmaceutical composition is prepared by compounding under an inert environment such as high purity nitrogen or argon. In a preferred embodiment, the pharmaceutical composition is compounded under a nitrogen environment with less than 2 ppm of oxygen.

In some embodiments, the pharmaceutical composition is prepared by compounding at temperatures between 20-25°C.

In a preferred embodiment, the solid LACE molecule is ground up into a fine powder. Preferably, the solid LACE molecule is ground up into a powder with no clumps. In an embodiment, the particle size will be less than 500 microns. In another preferred embodiment, the particle size will be less than 100 microns.

In a preferred embodiment, the pharmaceutical composition is prepared by initial de-aeration of the aqueous solution maintained at room temperature (20-25°C), then dissolution of the excipients in the solution, followed by adding the solid LACE slowly in parts under vigorous dissolution under nitrogen slow sparging.

In one embodiment, the pharmaceutical composition is stirred vigorously for 4 hours to 24 hours. In a preferred embodiment, the pharmaceutical composition is stirred vigorously from 4 hours to 8 hours. In another preferred embodiment, the pharmaceutical composition is stirred vigorously for 8 hours.

The pharmaceutical composition prepared by either method can have a shelf-stability of at least 3 months (e.g., 3 months, 6 months, 9 months, 1 year, or more than 1 year).

The pharmaceutical composition can also have favorable profiles of drug related degradant (e.g., total drug related impurities, or amount of a specific drug related impurity) following storage at 5 °C for a certain period of time. Analytical tools (e.g., HPLC) for measuring the amount of drug related degradant in a formulation are known.

Suitable biochemically acceptable energy source can be any of those known in the art. For example, the biochemical acceptable energy source can be any of those that can facilitate reduction by participating as an intermediate of energy metabolic pathways, particularly the glucose metabolic pathway. Non-limiting examples of suitable biochemically acceptable energy source include amino acids or derivative thereof (e.g., alanine, glycine, valine, leucine, isoleucine, 2-oxoglutarate, glutamate, and glutamine, etc.), a sugar or metabolites thereof (e.g., glucose, glucose-6-phosphate (G6P)), pyruvate (e.g., ethyl pyruvate), lactose, lactate, or derivatives thereof), a lipid (e.g., a fatty acid or derivatives thereof such as mono-, di-, and tri-glycerides and phospholipids), and others (e.g., NADH). Suitable amount of a biochemically acceptable energy source can be in the range of 0.01% to 5% (e.g., 0.05%, 0.1%, 0.2%, 0.5%, 1%, 2%, 3%, 4%, 5%, or any ranges based on these specified numeric values) by weight of the composition. In some embodiments, the biochemical energy source is ethyl pyruvate. In some embodiments, the biochemical energy source is alanine. In some embodiments, the amount of ethyl pyruvate or alanine is in the range of 0.05% to 5% (e.g., 0.05%, 0.1%, 0.2%, 0.5%, 1%, 2%, 3%, 4%, 5%, or any ranges based on these specified numeric values) by weight of the composition. In some embodiments, the amount of alanine is 0.5% by weight of the composition. In any of the embodiments described herein, the biochemically acceptable energy source is in an amount that is ophthalmically acceptable.

Suitable preservatives can be any of those known in the art. Non-limiting examples include benzalkonium chloride (BAC), cetrimonium, chlorobutanol, edetate disodium (EDTA), polyquaternium-1 (Polyquad®), polyhexamethylene biguanide (PHMB), stabilized oxychloro complex (PURITE®), sodium perborate, and SofZia®. Suitable amount of a preservative in the pharmaceutical composition can be in the range of 0.005% to 0.1% (e.g., 0.005, 0.01, 0.02%, 0.05%, 0.1%, or any ranges based on these specified numeric values) by weight of the composition. In some embodiments, the preservative is benzalkonium chloride. In some embodiments, the benzalkonium chloride is in the amount of 0.003% to 0.1% (e.g., 0.003, 0.01, 0.02%, 0.05%, 0.1%, or any ranges based on these specified numeric values) by weight of the composition. In some embodiments, the benzalkonium chloride is in the amount of 0.01% by weight of the composition. In any of the embodiments described herein, the preservative is in an amount that is ophthalmically acceptable. In some embodiments, the pharmaceutical composition is free of a preservative.

Suitable tonicity agents can be any of those known in the art. Non-limiting examples include sodium chloride, potassium chloride, mannitol, dextrose, glycerin, propylene glycol and mixtures thereof. Suitable amount of tonicity agent in the pharmaceutical composition is any amount that can achieve an osmolality of 200-460 mOsm (e.g., 260-360 mOsm, or 260-320 mOsm). In some embodiments, the pharmaceutical composition is an isotonic composition. In some embodiments, the amount of a tonicity agent (e.g., sodium chloride) is 0.1% to 5% (e.g., 0.1%, 0.5%, 1%, 2%, 3%, 4%, 5%, or any ranges based on these specified numeric values) by weight of the composition. In any of the embodiments described herein, the tonicity agent is in an amount that is ophthalmically acceptable.

Suitable surfactant can be any of those known in the art, including ionic surfactants and nonionic surfactants. Non-limiting examples of useful nonionic surfactants include polyoxyethylene fatty esters (e.g., polysorbate 80 [poly(oxyethylene)sorbitan monooleate], polysorbate 60 [poly(oxyethylene)sorbitan monostearate], polysorbate 40 [poly(oxyethylene)sorbitan monopalmitate], poly(oxyethylene)sorbitan monolaurate, poly(oxyethylene)sorbitan trioleate, or polysorbate 65 [poly(oxyethylene)sorbitan tristearate]), polyoxyethylene hydrogenated castor oils (e.g., polyoxyethylene hydrogenated castor oil 10, polyoxyethylene hydrogenated castor oil 40, polyoxyethylene hydrogenated castor oil 50, or polyoxyethylene hydrogenated castor oil 60), polyoxyethylene polyoxypropylene glycols (e.g., polyoxyethylene (160) polyoxypropylene (30) glycol [Pluronic F681], polyoxyethylene (42) polyoxypropylene (67) glycol [Pluronic P123], polyoxyethylene (54) polyoxypropylene (39) glycol [Pluronic P85], polyoxyethylene (196) polyoxypropylene (67) glycol [Pluronic F1271], or polyoxyethylene (20) polyoxypropylene (20) glycol [Pluronic L-441]), polyoxyl 40 stearate, sucrose fatty esters, and a combination thereof. In some embodiments, the surfactant is polysorbate 80. Suitable amount of surfactant in the pharmaceutical composition can be in the range of 0.01% to 5% (e.g., 0.05, 0.1, 0.2%, 0.5%, 1%, 2%, 3%, 4%, 5%, or any ranges based on these specified numeric values) by weight of the composition. In some embodiments, the surfactant is polysorbate 80, and the amount of polysorbate 80 is in the range of 0.05% to 5% (e.g., 0.05, 0.1, 0.2%, 0.5%, 1%, 2%, 3%, 4%, 5%, or any ranges based on these specified numeric values) by weight of the composition. In some embodiments, the amount of polysorbate 80 is 0.5% by weight of the composition. In any of the embodiments described herein, the surfactant is in an amount that is ophthalmically acceptable. However, in some embodiments, the pharmaceutical composition is free of a surfactant.

Suitable viscosity modifying agent can be any of those known in the art. Non-limiting examples include carbopol gels, cellulosic agents (e.g., hydroxypropyl methylcellulose), polycarbophil, polyvinyl alcohol, dextran, gelatin glycerin, polyethylene glycol, poloxamer 407, polyvinyl alcohol and polyvinyl pyrrolidone and mixtures thereof. Suitable amount of viscosity modifying agent can be in the range of 0.1% to 5% (e.g., 0.1%, 0.5%, 1%, 2%, 3%, 4%, 5%, or any ranges based on these specified numeric values) by weight of the composition. In any of the embodiments described herein, the viscosity modifying agent is in an amount that is ophthalmically acceptable. In some embodiments, the pharmaceutical composition is free of a viscosity modifying agent (e.g., a polymeric viscosity modifying agent such as hydroxypropyl methylcellulose).

In some embodiments, the pharmaceutical composition is characterized by one or more of the following:
(a) having a concentration of the lipoic acid choline ester from 0.1% to 10% (e.g., 0.1%, 1.0%, 1.5%, 3%, 4%, 5%, or any ranges between the specified numeric values) by weight of the composition;
(b) having a concentration of a preservative (e.g., benzalkonium chloride) of 0.003% to 0.1% (e.g., 0.01%) by weight of the composition;
(c) having a biochemical energy source (e.g., alanine) of 0.1% to 5% (e.g., 0.5%) by weight of the composition; and
(d) having a concentration of glycerol of 0.5% to 5% (e.g., 2.7%) by weight of the composition.

In some embodiments, the pharmaceutical composition consists essentially of 1-3% by weight of glycerin, 0.5% by weight of alanine, 0.005-0.01% by weight of benzalkonium chloride, 1-3% by weight of lipoic acid choline ester, and water, wherein the pH of the pharmaceutical composition is 4.3 to 4.7.

In one embodiment, the ophthalmic composition is dosed to each eye of the subject once daily, twice daily, thrice daily and four times daily.

In some embodiments, the invention also provides a system for storing a pharmaceutical composition comprising an active ingredient in an aqueous solution, wherein the active ingredient (e.g., lipoic acid choline ester or derivatives thereof) is susceptible to hydrolysis in the aqueous solution. In a preferred embodiment, the pharmaceutical composition is stored in a LDPE ophthalmic eye-dropper bottle, overlaid with nitrogen during the filling process, capped, then packed in a secondary mylar, gas-impermeable pouch containing an oxygen absorbent.

In another embodiment, the eye-dropper bottle or unit is polyethylene terephthalate (PET). In another embodiment, the eye-dropper bottle is constructed of a material that has low gas permeability.

In other embodiment, eye-dropper bottle can be constructed of any material that has a low gas permeability. In another embodiment, the eye-dropper bottle can be unit dose, filled by blow fill seal techniques.

In one embodiment, the pharmaceutical composition is stored at 2-5°C, for a period of 3 months to 2 years.

### VII. METHODS OF TREATMENT

The pharmaceutical compositions comprising lipoic acid choline ester or derivatives thereof (e.g., as described herein) can be employed in a method for treating or preventing a disease or disorder associated with oxidative damage. Diseases or disorders associated with oxidative damage are known.

In some embodiments, the invention provides a method of treating an ocular disease in a subject in need thereof, comprising administering to an eye of the subject a therapeutically effective amount of any of the pharmaceutical compositions described herein.

In some embodiments, the ocular diseases are presbyopia, dry eye, cataract, macular degeneration (including age-related macular degeneration), retinopathies (including diabetic retinopathy), glaucoma, or ocular inflammations. In some embodiments, the ocular disease is presbyopia.

Suitable amount of pharmaceutical compositions for the methods of treating or preventing an ocular disease herein can be any therapeutically effective amount. In some embodiments, the method comprises administering to the eye of the subject an amount of the pharmaceutical composition effective to increase the accommodative amplitude of the lens by at least 0.1 diopters (D) (e.g., 0.1, 0.2, 0.5, 1, 1.2, 1.5, 1.8, 2, 2.5, 3, or 5 diopters). In some embodiments, the method comprises administering to the eye of the subject 1-5 drops (about 40 uL per drop) of the pharmaceutical composition. In some embodiments, the eye of the subject is treated with the pharmaceutical composition 1, 2, 3, 4, 5, or more than 5 times a day, each time with 1-5 drops (about 40 µL per drop). In some embodiments, the lens or eye of the subject is treated with the pharmaceutical composition 1, 2, 3, 4, 5, or more than 5 drops each time. In some embodiments, the eye of the subject is treated with the pharmaceutical composition herein twice or three times per day, each time with 1 or 2 drops (about 40 uL per drop).

The methods include preventative methods that can be performed on patients of any age. The methods also include therapeutic methods that can be performed on patients of any age, particularly patients that are between 20-75 years of age.

The following examples are illustrative and do not limit the scope of the claimed embodiments.

### EXAMPLES

### Example 1

**Table 1: General Properties of Lipoic Acid Choline Ester (LACE)**

| | |
|---|---|
| Appearance: | |
| | |
| Solubility Profile: | >50 mg/mL in water |
| | > 4 mg/mL in acetonitrile |
| | |
| Solution pH | 7-7.5 |
| | |
| Log P | <2 |
| | |
| Specific rotation: | 70.3° |
| | |
| Optical rotation: | 0.338 at 25°C at 0.005g/mL in acetonitrile |
| | |
| Spectral properties: | UV λₘₐₓ 333 nm |
| | |
| Hygroscopicity: | Highly hygroscopic |
| | |
| Crystallinity: | Sharp crystalline melt transition not observed, mostly amorphous |
| | |
| Polymorphs: | Not known at this time |
| | |
| Particle size: | D₅₀: 100-200 mm |
| | |
| Melting/boiling range: | Thermal transition observed at 230-235°C |

### Example 2

### Kinetics of Micellar Species Correlated to the Mixing Time of LACE Process Solutions

The experiments described in this section demonstrate that the LACE micellar species stabilize and diminish over extended mixing times at 25°C. The results demonstrated that the reversible nature of these species characteristic of self-assembled systems such as micelles and micellar aggregates.

Micellar species formed by spontaneous self-assembly of molecules are driven by the total free energy of the equilibrated system. The experiment demonstrated the kinetics of achievement of that equilibrated state with longer durations of mixing.

### Objectives:

∘ Establish process-time bracket by establishing if growing micellar species has stabilized.
∘ Establish "holding time"

### Procedure:

∘ Two 200-g batches of 1.5% LACE were prepared at 25°C after vehicle was deoxygenated with bubbled nitrogen. Nitrogen was continually bubbled during dissolution of LACE.
∘ One batch was prepared using GMP Batch #2 (G2-14LAC) as is, with significant clumps, the other was prepared using a sample of G2-14LAC that had been ground into a fine powder using a mortar and pestle.
∘ After dissolution of the LACE and pH adjustment, the batches were stirred for 24 hours with a constant nitrogen overlay, maintaining dissolved oxygen at ∼1.6 ppm (vs. 8.2 ppm saturated solubility). At time-points of 1h, 3h, 4h, 6h, 8h, 9h, and 24h, about 5-15 mL was removed by syringe and sterile-filtered into eye-dropper bottles (5 mL/bottle), without nitrogen overlay in the bottle (apparatus was in use for the bulk batches).
∘ Samples of all time-points were diluted to 10 mg/g, and then injected for RP-HPLC analysis with ELSD detection within 30 minutes of removal from the bulk solution.
∘ After the 24-hour time-point, bulk solutions were sterile-filtered, and each divided into two ∼ 50 mL portions - one held at 5°C, the other 50-mL portion held at 25°C. All portions were overlaid with nitrogen blown into the vessel.
∘ At the end of the additional 24-hour hold time, each portion was filled into eye-dropper bottles with a nitrogen overlay in the bottle.

### Observations on Dissolution

∘ The clumped portion of G2-14LAC was added to formulation KW-LACE-01-86-1 over about 5 minutes, and some of the clumps required another 20 minutes to dissolve.
∘ The powdered G2-14LAC was added to formulation KW-LACE-01-86-2 over about 15 minutes, because each spatula-full aggregated into a thin raft of material floating on the surface, which did not disperse immediately. Therefore another portion was not added until previous portions were drawn into the vortex. Estimated time for any one portion to dissolve was about 10 minutes, and the whole process took approximately 25 minutes.

### Results

∘ A peak at RT = 8 minutes (correlated with the micellar species) was evident in both formulations from the first time-point taken.
∘ There were no consistent differences between the two batches in the % Area of the 8 min. peak (micellar species), though the 2nd batch, made with powdered LACE, had higher levels of the micellar species at some time-points.
∘ The %Area of the 8 min. peak was significantly reduced at 24 hours, as shown in the table below.
∘ Final pH was 4.54, for both batches.

**Table 2:**

| Kinetics of the Formation and De-Agglomeration of LACE Micellar Species with Extensive Mixing | | |
|---|---|---|
| | Percent Area of RT = 8 min. broad peak (LACE Micellar Species) | |
| | Total area of LACE and related peaks (Results are for 1^{st} injection from HPLC vial, unless noted) | |
| | **KW-LACE-01-86-1** | **KW-LACE-01-86-2** |
| | 1.5% LACE with clumps | 1.5% LACE ground up |
| Mixing Time (Hours) | % micellar species at 8 minutes | % micellar species at 8 minutes |
| 1 hour | 0.30% | 0.63% |
| 2 hours | 0.51% | 0.56% |
| 3 hours | 0.56% | 0.67% |
| 4 hours | 0.50% | 0.44% |
| 6 hours | 0.64%* | 0.70%* |
| 8 hours | 0.47% | 0.44% |
| 9 hours | 0.42% | 0.44% |
| 24 hours | 0.07% | 0.17% |
| 48 h (5°C hold) | Not detected | 0.09% |
| 48 h (25°C hold) | Not detected | Not detected |

The results demonstrate that LACE micellar species at 8.1 minute are minimized with extended mixing times. The peak at 8.1 minutes is diminished dramatically with longer mixing times.

Each of the solutions was also measured for degradants of lipoic acid choline ester. As mentioned earlier, the degradation mechanism of LACE is oxidative and hydrolytic, resulting in oxidized and hydrolyzed species.

**Table 3:**

| Impurity (Related Substances) Analysis of EV06 Ophthalmic Solution as a Function of Mixing | | | | | | | | | | | | | |
|---|---|---|---|---|---|---|---|---|---|---|---|---|---|
| KW-LACE-01-86-1: G2-14LAC with clumps | Time Point → | 1 hour | | 3 hours | | 4 hours | | 6 hours | | 8 hours | | 9 hours | |
| Related Compounds | Specification Individual: Report ≥ 0.1% (%Area) Total: Report | RRT 0.50 | 0.28% | RRT 0.50 | 0.37% | RRT 0.50 | 0.38% | RRT 0.50 | 0.43% | RRT 0.50 | 0.47% | RRT 0.50 | 0.49% |
| | | RRT 0.54 | 0.14% | RRT 0.54 | 0.16% | RRT 0.54 | 0.16% | RRT 0.54 | 0.17% | RRT 0.54 | 0.17% | RRT 0.54 | 0.18% |
| | | RRT 0.57 | 0.16% | RRT 0.57 | 0.18% | RRT 0.57 | 0.18% | RRT 0.57 | 0.19% | RRT 0.57 | 0.20% | RRT 0.57 | 0.20% |
| | | Lipoic Acid | 0.02% | LipoicAcid | 0.03% | LipoicAcid | 0.03% | LipoicAcid | 0.03% | LipoicAcid | 0.03% | LipoicAcid | 0.03% |
| | | RRT 1.61 | 0.17% | RRT 1.61 | 0.17% | RRT 1.61 | 0.16% | RRT 1.61 | 0.16% | RRT 1.61 | 0.16% | RRT 1.61 | 0.16% |
| | | **Total** | 0.77% | **Total** | 0.91% | **Total** | 0.91% | **Total** | 0.98% | **Total** | 1.02% | **Total** | 1.05% |
| KW-LACE-01-86-1: G2-14LAC with clumps | Time Point → | 24 hours | | 48 hours 5°C hold after first 24h | | 48 hours 25°C hold after first 24h | | | | | | | |
| Related Compounds | Specification Individual: Report ≥ 0.1% (%Area) Total:Report | RRT 0.50 | 0.65% | RRT 0.50 | 0.68% | RRT 0.50 | 0.89% | | | | | | |
| | | RRT 0.54 | 0.20% | RRT 0.54 | 0.17% | RRT 0.54 | 0.21% | | | | | | |
| | | RRT 0.57 | 0.25% | RRT 0.57 | 0.20% | RRT 0.57 | 0.27% | | | | | | |
| | | LipoicAcid | 0.04% | LipoicAcid | 0.05% | LipoicAcid | 0.06% | | | | | | |
| | | RRT 1.61 | 0.16% | RRT 1.61 | 0.17% | RRT 1.61 | 0.20% | | | | | | |
| | | **Total** | **1.31%** | **Total** | **1.27%** | **Total** | **1.61%** | | | | | | |

The data shows that the degradation products of LACE rise with extended mixing time. Thus, final process conditions for the compounding of EV06 Ophthalmic Solution involved a maximum of 8 hours to achieve a non-irritating solution with minimized degradants.

### Example 3

### Correlation of Mixing Temperature with Presence of Micellar LACE Species

The data shown in FIG. 4 is of a solution of LACE chloride formulated under argon and refrigerated conditions. The solution was extremely irritating to the ocular surface. The percent micellar species was 8-10% of the main LACE API Peak (micellar species denoted by arrow, at retention time 7.9-8.1 minutes), a concentration that is normally not observed in solutions mixed at room temperature.

### Example 4

### Correlation of the Clumps to the Formation of Micellar LACE Species

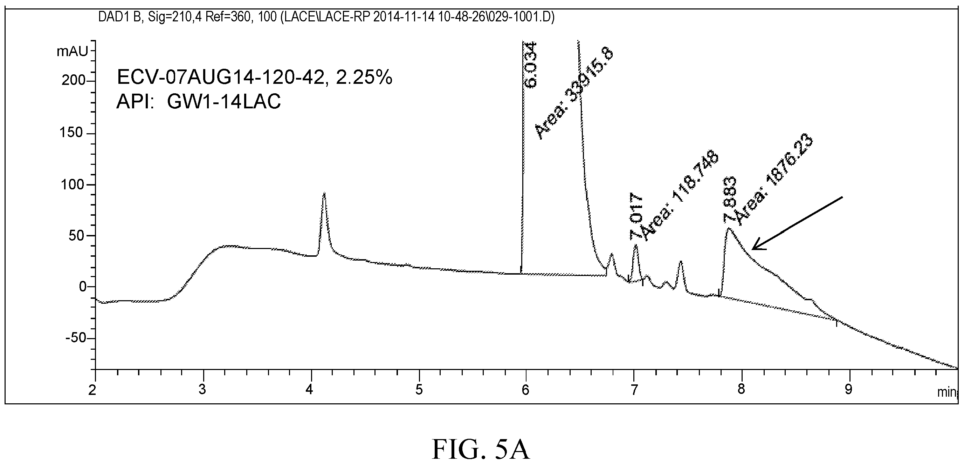

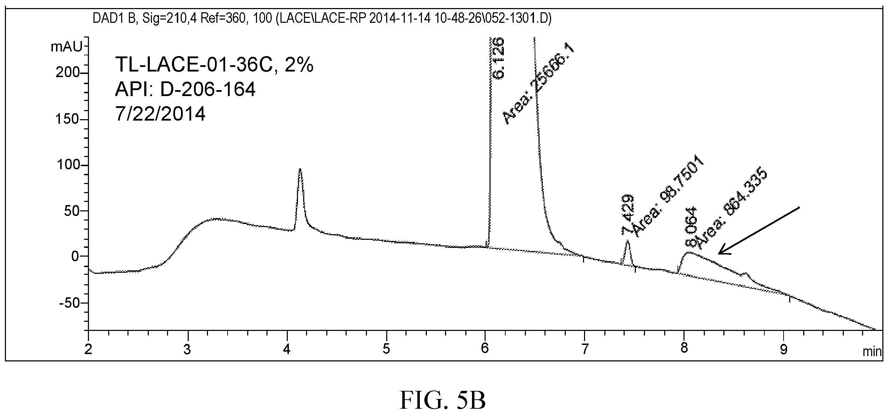

FIG. 5A is a RP-HPLC chloride chromatogram of EV06 Ophthalmic Solution prepared from a LACE batch that had solid "clumps". The solution prepared from this lot of API (active pharmaceutical ingredient, solid LACE drug substance) showed a higher percentage (10-15%) of the micellar LACE species (shown with an arrow) than solutions prepared from a lot of API that was powdery (FIG. 5B). Thus, while both solutions looked completely dissolved, the solution formulated from non-clumped API had a lower concentration of micellar LACE species. When correlated to ocular irritation, the solution shown in FIG. 5A had higher scores for irritation in a rabbit model. This led to incorporation of de-clumping procedures to render powdered material, prior to compounding.

### Example 5

### Compatibility Studies of Excipients with LACE

### SUMMARY

The purpose of these experiments was to tease out possible destabilizing variables in the formulation, through systematic variations in formulation composition and microenvironment (such as pH). Lipoic acid, and any derivatives of lipoic acid would be subject to degradation and polymerization in heat, light and oxygen, leading to opening of the dithiolane ring. Thus, presence of excipients that can induce oxidative free radical scission could be destabilizing factors. The formulation grids 1 and 2, systematically investigated the effect of excipients already present in the formulation, as possible destabilizing factors.

The formulations for LACE in these experiments contains the drug substance, alanine, glycerin, benzalkonium chloride in purified water, with 1N sodium hydroxide, or 1N hydrochloride acid added to achieve a pH between 4.4-4.6 and an osmolality of 290-300 mOsm/kg. The experiments described in this document were compatibility studies to identify excipients that could stabilize LACE ophthalmic solutions.

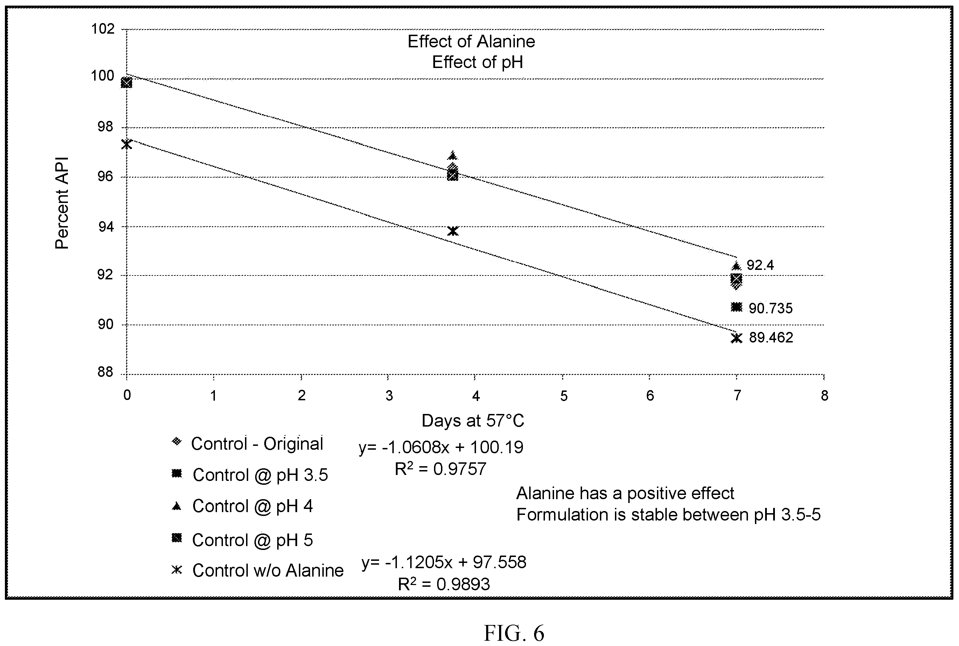

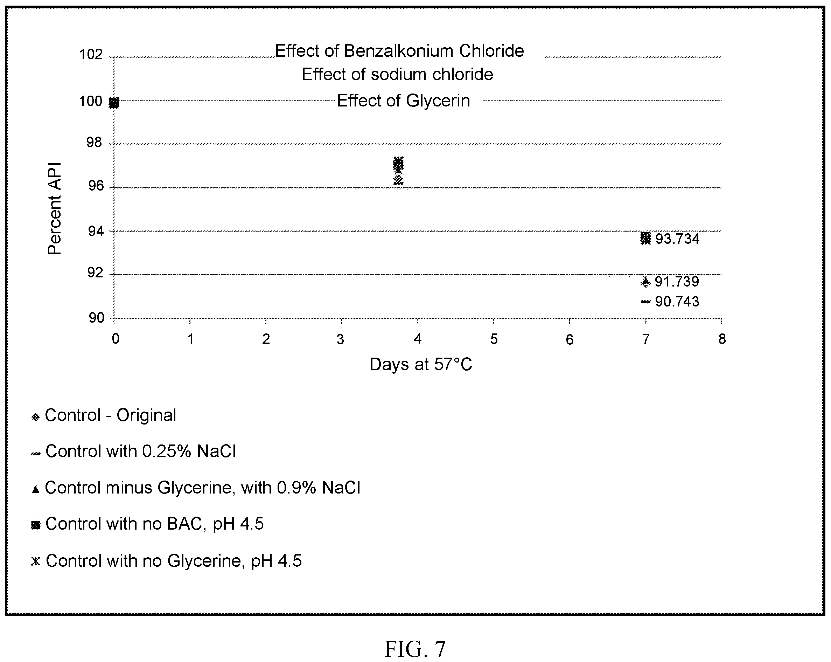

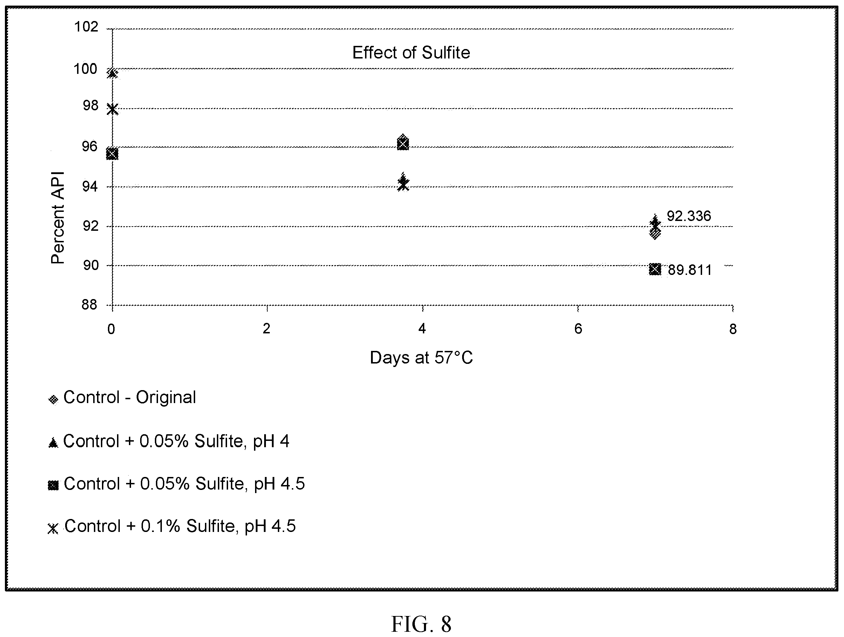

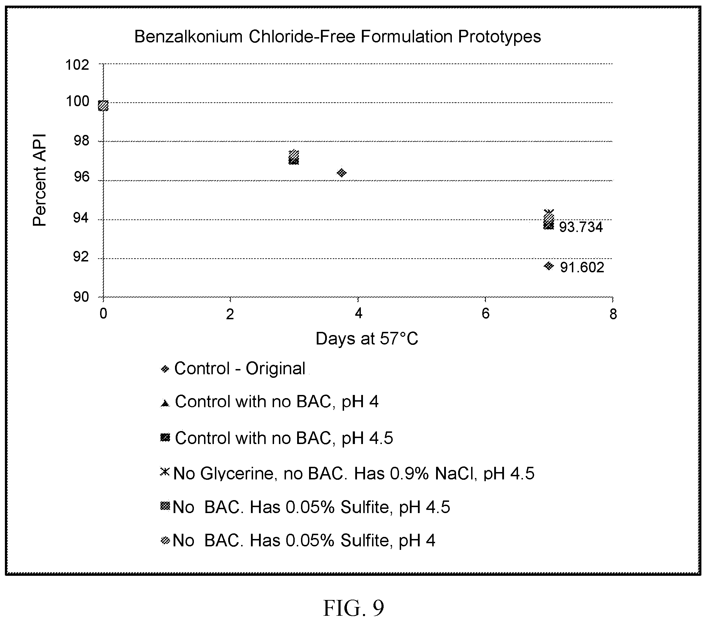

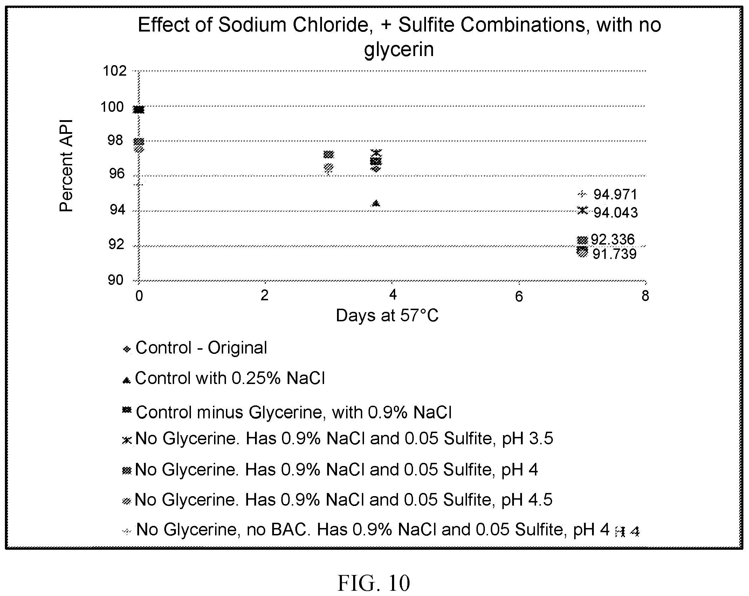

Formulation Grid#1 tested the following variables given in (a)-(e). The formulations were prepared in a nitrogen-flushed glove box and sterile filtered. All formulations were tested under accelerated conditions at 57°C and tested by HPLC for assay and impurities at T=0, 3.5 days and 7 days. A total of 19 formulations were tested in Grid#1.
(a) **Effect of pH:** Formulations were prepared at pH 3.5, 4 and 5, and compared with the control formulation at pH 4.5. As shown in FIG. 6, the rate of degradation of LACE was equivalent at all pH levels in the range 3.5-5.
(b) **Effect of Alanine:** The role of alanine in the formulation was deduced, by comparison of rates of degradation with the original formulation (control). As shown in FIG. 6, the absence of alanine appeared to accelerate the rate of degradation of LACE. Thus, Alanine is a critical excipient in EV06 Ophthalmic formulations.
(c) **Effect of Benzalkonium Chloride and Glycerin:** It was hypothesized that peroxides contained in glycerin can catalyze oxidation; similarly, it was hypothesized that BAC could destabilize the drug substance, due to free radical scission and subsequent oxidation. As seen in FIG. 7, the benzalkonium chloride-free formulation was substantially more stable than the control. The glycerin-free prototype was also more stable than the control. Additionally, sodium chloride added into the formulation (instead of glycerol, to adjust osmolality) appeared to have a destabilizing effect (also shown in FIG. 7). In another experiment with various combinations of glycerin, sodium chloride, sulfite and pH with all variations being benzalkonium chloride-free, it was remarkable that all of the benzalkonium chloride-free formulations were more stable than the control (FIG. 5). The experiments in FIG. 7 and 9 demonstrate that eliminating benzalkonium chloride in LACE may stabilize the formulation. For EV06 Ophthalmic compositions, minimizing benzalkonium chloride content to 50 ppm may have a major stabilizing factor. Sodium chloride demonstrated a destabilizing effect, thus glycerol was deemed more suitable as a tonicity agent in final EV06 compositions.
(d) **Effect of Sulfite:** Various experiments were performed with sulfite (FIG.8), with combinations of various levels of sulfite. Sulfite was added to the formulation as an anti-oxidant (FIG.8), at various pH levels (4, 4.5) and concentrations. The presence of sulfite did not appear to substantially improve the stability the LACE. It was not clear if a deleterious effect was present, since 0.1% sulfite in the formulation was equivalent to the control.
(e) **Effect of glycerin:** The effect of glycerin was investigated in various formulation combinations, by the systematic elimination of glycerin. As shown in FIG. 7 and 10, the glycerin-free combinations appeared to be more stable than control. However, due to the high destabilizing effect of sodium chloride, glycerin was selected as the critical excipient for tonicity adjustment.
(f) **Effect of Buffer:** Various buffered compositions were tested. Acetate buffer and acetate + boric acid appeared to stabilize the formulation.

### EXPERIMENTALS

a) HPLC Method Setup: The HPLC assay consisted of a 50 minute mobile phase gradient made up of (A) 0.05M sodium phosphate monobasic, 0.005M 1-heptane sulfonic acid sodium salt, 0.2% v/v triethylamine, adjusted to pH 4.5 with phosphoric acid; and (B) acetonitrile. The analytical column used is a YMC Pack ODS AQ (4.6x250 mm, 5 µm, 120 Å), P/N AQ125052546WT; the analytical detection wavelength is 225 nm.
b) FORMULATIONS

Formulations were prepared with extensive care to ensure that the LACE API was not exposed to oxygen or heat. The API was aliquotted into clean glass vials under an inert N₂ atmosphere inside of glove bag, and stored wrapped in tinfoil in a -20°C freezer until use. The formulations were prepared with high purity excipients, and sterile glassware. All excipients were pre-prepared in stock solutions and were mixed together before the addition of API & final pH adjustments. The formulations are tabulated in Appendix A.

### II. RESULTS AND DISCUSSION

FIG. 6 is a plot of % LACE API versus Days at 57°C (over T=0, 3.5 days and 7 days), systematically comparing formulations that were prepared at pH 3.5, 4, 4.5 (original), 5 and control w/o alanine. Even at T=0, the formulation w/o alanine had degraded considerably in API content. As seen in FIG. 6, the formulations were equivalent under these conditions at pH 3.5-5. FIG. 7 is a plot of formulations comparing the following variables: (a) Control (original) versus Control + 0.25% sodium chloride, Control + 0.25% sodium chloride, w/o glycerin, (b) Control (original) versus control, w/o benzalkonium chloride, (c) Control (original) versus original formulation without glycerin.

As seen in FIG. 7, addition of sodium chloride to the original formulation did not stabilize the formulation.

FIG. 8 shows the effect of sulfite on LACE stability at 57°C. Sulfite-containing formulations were prepared at concentrations 0.05% sulfite (■, ▲) and 0.1% sulfite (x) at pH 4 and 4.5. Addition of sulfite did not stabilize the original formulation (◆).

FIG. 9 further explores the potentially stabilizing effect of eliminating benzalkonium chloride. All formulation variations without benzalkonium chloride were superior to the control original formulation (pH 4.5). Formulation variations were BAC-free compositions at pHs 4, 4.5 (▲,■), no glycerin/no BAC + 0.9% sodium chloride (x), no BAC + 0.05% sulfite at pHs 4 and 4.5 (●, ■).

FIG. 7 and 10 compares the effect of eliminating glycerin, in various compositions, as a function of pH, sulfite and sodium chloride. The no-glycerin, no-BAC formulation in the presence of sodium chloride and sulfite (+) and the no-glycerin, w/ BAC formulation (*) were superior to the original formulation (◆).

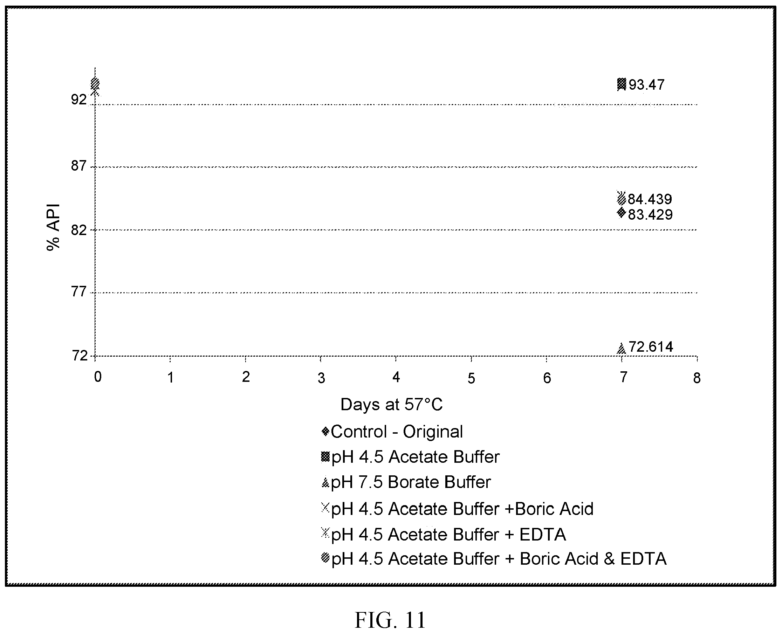

FIG. 11 explored the use of various buffered compositions on LACE stability. The original formulation (pH 4.5) was compared with acetate buffer compositions (■, x, *, ●) and borate at pH 7.5. Sodium edetate added as an anti-oxidant did not stabilize the formulation. Acetate buffer and acetate buffer + boric acid appeared to be superior to the control formulation.

To summarize, elimination of benzalkonium chloride appeared to enhance stability consistently. Elimination of glycerol may be a positive step as well. Glycerol is known to have residual presence of formaldehyde which occasionally leads to degradation of API. Interestingly, addition of edetate or sulfite did not have a positive effect. Another anti-oxidant such as sodium ascorbate may have a positive effect.

**Table 4:**

| Compatibility Experiment Formulations | | | | |
|---|---|---|---|---|
| Formulation # 1 | | | | |
| **Ingredient** | **Desired % w/w** | **grams needed** | **grams used** | **Act. w/w %** |
| Lipoic Acid Choline Ester (LACE) | 1 | 0 | 0.1849 | 0.9253 |
| Alanine | 0.5 | 0 | 2.0254 | 0.5068 |
| Glycerine | 0.1 | 0 | 0.401 | 0.0996 |
| Benzalkonium Chloride | 0.01 | 0 | 0.0378 | 0.0094 |
| Ultrapure Water (1st addition) | 75 | 15 | 12.338 | 61.7409 |
| 1N NaOH | Used to adjust to water desired pH before final addition | | 0.0815 | 0.0143 |
| 1N HCl | | | | |
| Ultrapure Water (2nd addition) | qs | qs | 4.9149 | 24.5948 |
| Total | 100 | 20 | 19.9835 | |
| **Desired pH** | **Final pH** | **mOsm/L** | | |
| 4.5 | 4.55 | | | |
| | | | | |

| Formulation # 2 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1977 | 0.9923 |
| Alanine | 0 | 0 | 0 | 0.0000 |
| Glycerine | 0.1 | 0 | 0.4009 | 0.0998 |
| Benzalkonium Chloride | 0.01 | 0 | 0.0386 | 0.0097 |
| Ultrapure Water (1st addition) | 75 | 15 | 14.4079 | 72.3176 |
| 1N NaOH | Used to adjust to water desired pH before final addition | | 0.1185 | 0.0209 |
| 1N HCl | | | | |
| Ultrapure Water (2nd addition) | qs | qs | 4.7595 | 23.8894 |
| Total | 100 | 20 | 19.9231 | |
| **Desired pH** | **Final pH** | **mOsm/L** | | |
| 4.5 | 4.48 | | | |
| | | | | |

| Formulation # 3 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1993 | 0.9935 |
| Alanine | 0.5 | 0 | 2.0227 | 0.5042 |
| Glycerine | 0.1 | 0 | 0.4031 | 0.0997 |
| Benzalkonium Chloride | 0.01 | 0 | 0.0409 | 0.0102 |
| Ultrapure Water (1st addition) | 75 | 15 | 12.414 | 61.8862 |
| 1N NaOH | Used to adjust to water desired pH before final addition | | 0.0653 | 0.0114 |
| 1N HCl | | | | |
| Ultrapure Water (2nd addition) | qs | qs | 4.9141 | 24.4977 |
| Total | 100 | 20 | 20.0594 | |
| **Desired pH** | **Final pH** | **mOsm/L** | | |
| 3.5 | 3.59 | | | |

| Formulation # 4 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.2036 | 1.0357 |
| Alanine | 0.5 | 0 | 2.021 | 0.5141 |
| Glycerine | 0.1 | 0 | 0.4012 | 0.1013 |
| Benzalkonium Chloride | 0.01 | 0 | 0.0402 | 0.0102 |
| Ultrapure Water (1st addition) | 75 | 15 | 12.3097 | 62.6215 |
| 1N NaOH | Used to adjust to desired pH before final water addition | | 0.0289 | 0.0052 |
| 1N HCl | | | | |
| Ultrapure Water (2nd addition) | qs | qs | 4.6527 | 23.6691 |
| Total | 100 | 20 | 19.6573 | |
| **Desired pH** | **Final pH** | **mOsm/L** | | |
| 4 | 4.07 | | | |
| | | | | |

| Formulation # 5 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.2368 | 1.1813 |
| Alanine | 0.5 | 0 | 2.0239 | 0.5048 |
| Glycerine | 0.1 | 0 | 0.4016 | 0.0994 |
| Benzalkonium Chloride | 0.01 | 0 | 0.041 | 0.0102 |
| Ultrapure Water (1st addition) | 75 | 15 | 12.3029 | 61.3746 |
| 1N NaOH | Used to adjust to desired pH before final water addition | | 0.0129 | 0.0023 |
| 1N HCl | | | | |
| Ultrapure Water (2nd addition) | qs | qs | 5.0265 | 25.0753 |
| Total | 100 | 20 | 20.0456 | |
| **Desired pH** | **Final pH** | **mOsm/L** | | |
| 5 | 5.01 | | | |
| | | | | |

| Formulation # 6 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.2013 | 1.0040 |
| Alanine | 0.5 | 0 | 2.0216 | 0.5041 |
| Sodium Chloride | 0.25 | 0 | 0.5282 | 0.2635 |
| Glycerine | 0.1 | 0 | 0.4025 | 0.0996 |
| Benzalkonium Chloride | 0.01 | 0 | 0.04 | 0.0100 |
| Ultrapure Water (1st addition) | 75 | 15 | 11.8086 | 58.8963 |
| 1N NaOH | Used to adjust to desired pH before final water addition | | 0.0208 | 0.0036 |
| 1N HCl | | | | |
| Ultrapure Water (2nd addition) | qs | qs | 5.0268 | 25.0716 |
| Total | 100 | 20 | 20.0498 | |
| **Desired pH** | **Final pH** | **mOsm/L** | | |
| 4 | 4.01 | | | |

| Formulation # 7 | | | | |
|---|---|---|---|---|
| **Ingredient** | **Desired w/w** | **grams needed** | **grams used** | **Act. w/w %** |
| Lipoic Acid Choline Ester (LACE) | 1 | 0 | 0.2004 | 0.9999 |
| Alanine | 0.5 | 0 | 2.0209 | 0.5042 |
| Sodium Chloride | 0.9 | 0 | 1.8409 | 0.9186 |
| Glycerine | 0 | 0 | 0 | 0.0000 |
| Benzalkonium Chloride | 0.01 | 0 | 0.0402 | 0.0100 |
| Ultrapure Water (1st addition) | 75 | 15 | 10.9219 | 54.4961 |
| 1N NaOH | Used to adjust to desired pH before final water addition | | 0.009 | 0.0016 |
| 1N HCl | | | | |
| Ultrapure Water (2nd addition) | qs | qs | 5.0083 | 24.9895 |
| Total | 100 | 20 | 20.0416 | |
| **Desired pH** | **Final pH** | **mOsm/L** | | |
| 4 | 4.12 | | | |
| | | | | |

| Formulation # 8 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1956 | 0.9784 |
| Alanine | 0.5 | 0 | 2.022 | 0.5057 |
| Sodium Sulfite | 0.05 | 0 | 0.2068 | 0.0517 |
| Glycerine | 0.1 | 0 | 0.4005 | 0.0994 |
| Benzalkonium Chloride | 0.01 | 0 | 0.0401 | 0.0100 |
| Ultrapure Water (1st addition) | 75 | 15 | 12.16 | 60.8219 |
| 1N NaOH | Used to adjust to desired pH before final water addition | | 0.1008 | 0.5042 |
| 1N HCl | | | | |
| Ultrapure Water (2nd addition) | qs | qs | 4.867 | 24.3438 |
| Total | 100 | 20 | 19.9928 | |
| **Desired pH** | **Final pH** | **mOsm/L** | | |
| 4 | 4.03 | | | |
| | | | | |

| Formulation # 9 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.2205 | 1.0953 |
| Alanine | 0.5 | 0 | 2.027 | 0.5035 |
| Sodium Sulfite | 0.05 | 0 | 0.2062 | 0.0512 |
| Glycerine | 0.1 | 0 | 0.3996 | 0.0985 |
| Benzalkonium Chloride | 0.01 | 0 | 0.0392 | 0.0097 |
| Ultrapure Water (1st addition) | 75 | 15 | 12.1982 | 60.5944 |
| 1N NaOH | Used to adjust to desired pH before final water addition | | 0.0995 | 0.4943 |
| 1N HCl | | | | |
| Ultrapure Water (2nd addition) | qs | qs | 4.9407 | 24.5429 |
| Total | 100 | 20 | 20.1309 | 100.0000 |
| **Desired pH** | **Final pH** | **mOsm/L** | | |
| 4.5 | 4.03 | | | |

| Formulation # 10 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1973 | 0.9873 |
| Alanine | 0.5 | 0 | 2.0249 | 0.5066 |
| Sodium Sulfite | 0.1 | 0 | 0.4136 | 0.1035 |
| Glycerine | 0.1 | 0 | 0.4013 | 0.0996 |
| Benzalkonium Chloride | 0.01 | 0 | 0.041 | 0.0102 |
| Ultrapure Water (1st addition) | 75 | 15 | 11.9708 | 59.9031 |
| 1N NaOH | Used to adjust to desired pH before final water addition | | 0.1921 | 0.9613 |
| 1N HCl | | | | |
| Ultrapure Water (2nd addition) | qs | qs | 4.7426 | 23.7325 |
| Total | 100 | 20 | 19.9836 | |
| **Desired pH** | **Final pH** | **mOsm/L** | | |
| 4.5 | 4.35 | | | |

| Formulation # 11 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.2097 | 0.9746 |
| Alanine | 0.5 | 0 | 2.0437 | 0.4749 |
| Sodium Chloride | 0.9 | 0 | 1.8022 | 0.8376 |
| Sodium Sulfite | 0.05 | 0 | 0.2116 | 0.0492 |
| Glycerine | 0 | 0 | 0 | 0.0000 |
| Benzalkonium Chloride | 0.01 | 0 | 0.0384 | 0.0089 |
| Ultrapure Water (1st addition) | 75 | 15 | 17.0628 | 79.3025 |
| IN NaOH | Used to adjust to desired pH before final water addition | | 0.1477 | 0.0241 |
| 1N HCl | | | | |
| Ultrapure Water (2nd addition) | qs | qs | | |
| Total | 100 | 20 | 21.5161 | |
| **Desired pH** | **Final pH** | **mOsm/L** | | |
| 3.5 | 3.52 | | | |

| Formulation # 12 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1953 | 0.9703 |
| Alanine | 0.5 | 0 | 2.0402 | 0.5068 |
| Sodium Chloride | 0.9 | 0 | 1.7998 | 0.8943 |
| Sodium Sulfite | 0.05 | 0 | 0.2135 | 0.0531 |
| Glycerine | 0 | 0 | 0 | 0.0000 |
| Benzalkonium Chloride | 0.01 | 0 | 0.0423 | 0.0105 |
| Ultrapure Water (1st addition) | 75 | 15 | 15.6996 | 78.0031 |
| IN NaOH | Used to adjust to desired pH before final water addition | | 0.1362 | 0.0238 |
| 1N HCl | | | | |
| Ultrapure Water (2nd addition) | qs | qs | | |
| Total | 100 | 20 | 20.1269 | |
| **Desired pH** | **Final pH** | **mOsm/L** | | |
| 4 | 4 | | | |

| Formulation # 13 | | | | |
|---|---|---|---|---|
| **Ingredient** | **Desired % w/w** | **grams needed** | **grams used** | **Act. w/w** |
| Lipoic Acid Choline Ester (LACE) | 1 | 0 | 0.1997 | 0.9946 |
| Alanine | 0.5 | 0 | 2.0386 | 0.5077 |
| Sodium Chloride | 0.9 | 0 | 1.8033 | 0.8982 |
| Sodium Sulfite | 0.05 | 0 | 0.2152 | 0.0536 |
| Glycerine | 0 | 0 | 0 | 0.0000 |
| Benzalkonium Chloride | 0.01 | 0 | 0.0397 | 0.0099 |
| Ultrapure Water (1st addition) | 75 | 15 | 15.6858 | 78.1239 |
| 1N NaOH | Used to adjust to desired pH before final water addition | | 0.0958 | 0.0168 |
| 1N HCl | | | | |
| Ultrapure Water (2nd addition) | qs | qs | | |
| Total | 100 | 20 | 20.0781 | |
| **Desired pH** | **Final pH** | **mOsm/L** | | |
| 4.5 | 4.48 | | | |

| Formulation # 14 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.1976 | 0.9873 |
| Alanine | 0.5 | 0 | 2.0425 | 0.5103 |
| Glycerine | 0.1 | 0 | 0.4158 | 0.1031 |
| Benzalkonium Chloride | 0 | 0 | 0 | 0.0000 |
| Ultrapure Water (1st addition) | 75 | 15 | 17.3172 | 86.5237 |
| 1N NaOH | Used to adjust to desired pH before final water addition | | 0.0413 | 0.0073 |
| 1N HCl | | | | |
| Ultrapure Water (2nd addition) | qs | qs | | |
| Total | 100 | 20 | 20.0144 | |
| **Desired pH** | **Final pH** | **mOsm/L** | | |
| 4 | 4.05 | | | |

| Formulation # 15 | | | | |
|---|---|---|---|---|
| **Ingredient** | **Desired % w/w** | **grams needed** | **grams used** | **Act. w/w %** |
| Lipoic Acid Choline Ester (LACE) | 1 | 0 | 0.215 | 1.0757 |
| Alanine | 0.5 | 0 | 2.0453 | 0.5117 |
| Glycerine | 0.1 | 0 | 0.4078 | 0.1012 |
| Benzalkonium Chloride | 0 | 0 | 0 | 0.0000 |
| Ultrapure Water (1st addition) | 75 | 15 | 17.2993 | 86.5545 |
| 1N NaOH | Used to adjust to desired pH before final water addition | | 0.0192 | 0.0034 |
| 1N HCl | | | | |
| Ultrapure Water (2nd addition) | qs | qs | | |
| Total | 100 | 20 | 19.9866 | |
| **Desired pH** | **Final pH** | **mOsm/L** | | |
| 4.5 | 4.52 | | | |

### Example 6:

### Correlation of Ocular Irritation with Percent Micellar LACE Species

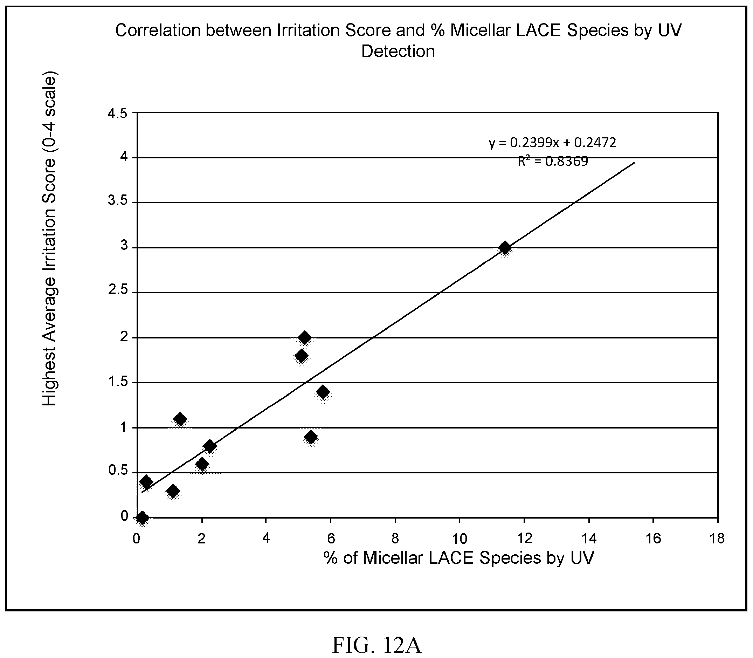

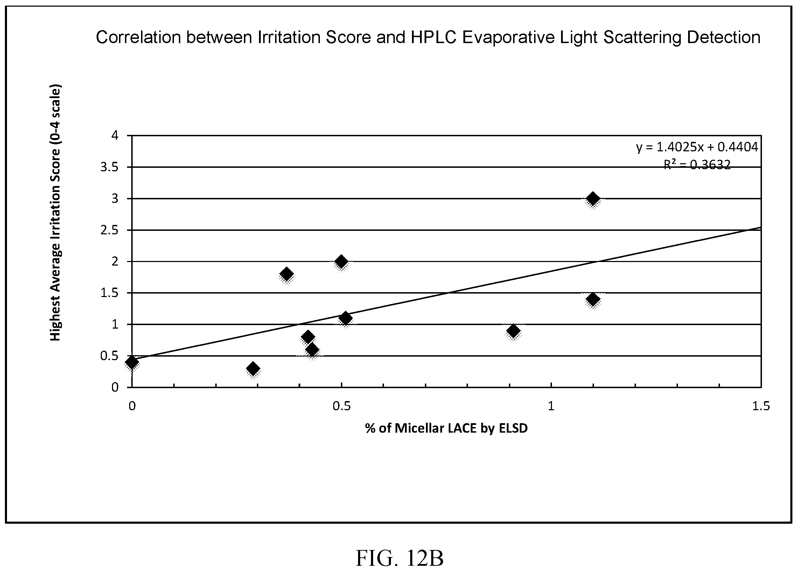

FIG. 12A and 12B generally provide a snapshot of the correlation of irritation to the micellar LACE species over a number of batches compounded.

### Example 7

### Method of Adjustment of Osmolality with Glycerol

The requisite osmolality range for drug-containing formulations and placebo is 280-320 mOsm/kg. Preferably, all LACE formulations need to be within 290-310 mOsm/Kg.

Since LACE has contributions to osmolality, each formulation will have varying concentrations of glycerol to achieve the requisite osmolality.

### I. Summary: Final Adjusted Compositions

**Table 5:**

| Final Compositions of EV06 Ophthalmic Solutions with Adjusted Glycerol Concentrat ions | | | | |
|---|---|---|---|---|
| LACE Conc. | Glycerol (%) | Alanine (%) | Benzalkonium Chloride (%) | Actual Total Osmolality (mOsm/kg) |
| 0% | 2.07% | 0.5% | 0.005% | 295 |
| 1% | 1.56% | 0.5% | 0.005% | 299 |
| 2% | 1.07% | 0.5% | 0.005% | 296 |
| 2.5% | 0.80% | 0.5% | 0.005% | 302 |
| 3.0% | 0.53% | 0.5% | 0.005% | 308 |

### II. Experimental Detail:

### A. Glycerol-containing Placebos

A series of placebos was prepared. All placebos, and the LACE-containing solutions that were subsequently prepared, contained the following, with varying amounts of Glycerol:
- 0.5% (5 mg/g) Alanine
- 0.005% (0.05 mg/g) Benzalkonium Chloride
- Small amounts of 1 N Sodium Hydroxide, 1 N Hydrochloric Acid, to adjust pH to 4.5
- Water for Injection (added for final weight)

**Table 6:**

| Glycerol-containing Placebo (Effect of Glycerol Concentration) | |
|---|---|
| Glycerol Percent | Osmolality (mOsm/kg) |
| 0.5% | 114 |
| 1.0% | 172 |
| 1.5% | 233 |
| 2.0% | 292 |
| 2.5% | 354 |

### B. LACE-Containing Formulations

Based on glycerol osmolality standard curve above (see Table 6) and Applicant's observations that showed an additional 44-55 mOsm/kg (average of 48 mOsm/kg) for every 1% LACE, a series of solutions was prepared to confirm the actual osmotic contribution of LACE. Target for Total Osmolality was 300 mOsm/kg.

**Table 7:**

| Glycerol Concentrations for EV06 Compositions | | | |
|---|---|---|---|
| LACE Conc. | Target Osmolality w/o LACE | Glycerol % for target Osm. | Actual Total Osmolality (mOsm/kg) |
| 0% | 300 | 2.06% | 295 |
| 1% | 250 | 1.64% | 308 |
| 2% | 203 | 1.25% | 324 |

These data indicate that the effect of LACE on osmolality is somewhat greater than expected, on the order of 57-60 mOsm/kg for every 1%. Accordingly, a full series of solutions was prepared with slightly altered target osmolalities for the solutions without LACE, and therefore different target glycerol contents. All solutions were prepared using the same Alanine/Benzalkonium Chloride, pH 4.5 stock solution used in the placebos, so that the final composition was consistently:
0.5% (5 mg/g) Alanine
0.005% (0.05 mg/g) Benzalkonium Chloride
Small amounts of 1 N Sodium Hydroxide, 1 N Hydrochloric Acid, to adjust pH to 4.5
Water for Injection (added to final weight of 5.0 g per formulation)

**Table 8:**

| Adjustment of Osmolality of EV06 Compositions | | | |
|---|---|---|---|
| LACE Conc. (Actual %) | Target Osmolality w/o LACE | Actual Glycerol % used for target Osm. | Actual Total Osmolality (mOsm/kg) |
| 0% | 300 | 2.06% | 295 |
| 1% (1.01%) | 242 | 1.56% | 299 |
| 2% (2.04%) | 180 | 1.06% | 296 |
| 2.5% (2.51%) | 150 | 0.80% | 302 |
| 3.0% (2.94%) | 120 | 0.56% | 308 |

### C. Sterile Preparations

Based on these experimental results, sterile filtered 10.0-g batches of each formulation were prepared, with the following target compositions, and packaged into sterile eye dropper bottles (2 mL per bottle):

**Table 9:**

| Final Composition Grid of EV06 Compositions | | | |
|---|---|---|---|
| LACE Conc. | Glycerol (%) | Alanine (%) | Benzalkonium Chloride (%) |
| 0% | 2.07% | 0.5% | 0.005% |
| 1% | 1.56% | 0.5% | 0.005% |
| 2% | 1.07% | 0.5% | 0.005% |
| 2.5% | 0.80% | 0.5% | 0.005% |
| 3.0% | 0.53% | 0.5% | 0.005% |

### Example 8

### Method of Preparation of LACE Pharmaceutical Compositions

A method of preparing LACE pharmaceutical composition is as follow:
∘ At room temperature, Water for Injection (WFI) at 80% of batch weight is added to glass compounding vessel. The water is purged with nitrogen to achieve ≤10 ppm oxygen.
∘ Stepwise, alanine, glycerin, and BAC, are added, and mixed until dissolved.
∘ The pH is adjusted to 4.4 - 4.6 with HCl or NaOH.
∘ LACE is ground in a mortar and pestle under nitrogen to de-clump and slowly added while mixing.
∘ Deoxygenated Water for Injection is added to achieve final batch target weight.
∘ Batch is mixed for a total of 8 hours to ensure complete dispersion and dissolution.
∘ The pH may be adjusted to 4.4 - 4.6 with NaOH or HCl if needed.
∘ Osmolality may adjusted to 290-310 with glycerol if needed.
∘ After 8 hours of mixing, EV06 bulk drug product solution is aseptically filtered through a capsule SHC 0.5/0.2 µm sterilizing filter into a holding bag.
∘ The bulk product solution in the holding bag is kept at 5°C by refrigeration or ice bath.
∘ Filter bubble point test is performed to ensure the integrity of the filter.
∘ Sterile filtered bulk solution is aseptically transferred to the Class 100 room and filled into pre-sterilized bottles.
∘ Sterile tips and caps are applied to the bottles under nitrogen overlay.
∘ Sealed bottles are transferred to trays, which are bagged with a nitrogen purge and immediately transferred to 5°C storage.

### Example 9

### Stability Studies of LACE Formulations

Early formulation prototypes contained sodium edetate and 0.01% benzalkonium chloride. Stability studies with and without these excipients demonstrated that sodium edetate did not stabilize LACE. Presence of excess benzalkonium chloride slightly destabilized the drug. Thus, the final formulation contains no sodium edetate and 0.005% benzalkonium chloride. Through microbiological testing, 0.004% benzalkonium chloride in the current formulation composition was shown to be effective as a preservative in the drug product.

In an effort to stabilize the drug formulation further, systematic stability studies (5°C, 25°C and 40°C) on mid-scale R&D batches were undertaken with bottled EV06 Ophthalmic Solution in the presence and absence of oxygen scavenging packets contained in zip-lock, vapor impermeable foil pouches. Bottles of product stored at 5°C in the presence of an oxygen scavenging packet sealed in re-sealable foil pouches demonstrated stability at 12 months.

Additional precautions were implemented throughout the development process to stabilize the final formulation from degradation due to exposure to environmental oxygen and non-refrigerated conditions. Handling of the drug substance under nitrogen (exclusion of oxygen and minimization of moisture) and compounding under a nitrogen blanket were implemented to minimize exposure to oxygen. After compounding, the product is filled into a vapor impermeable holding bag and stored under refrigerated conditions until bottling ensues. The holding bag containing the bulk solution is kept cold during filling. A nitrogen blanket is placed over the drug solution in each bottle, to minimize oxygen exposure.

**Table 10: Stability of EV06 Ophthalmic Solution (3.0%)**

| **Stability Table for EV06 Ophthalmic Solution, 3.0%** | | | | | | | | | |
|---|---|---|---|---|---|---|---|---|---|
| Container: Polyethylene dropper bottle, 6 cc | | | | Closure: Dropper Tip and Cap | | | | | |
| Secondary Container: Foil Pouch | | | | Oxygen Adsorbent: Oxygen Adsorbent Packet present | | | | | |
| **Test** | **Method** | **Acceptance Criteria** | **T=0** | **2 Weeks** | **1 Month** | **2 Month** | **3 Month** | **6 Month** | **12 Month** |
| **5°C** | | | | | | | | | |
| Appearance | ATM-1095 | Clear, pale yellow to yellow solution essentially free of foreign or particulate matter | Conforms | Conforms | Conforms | Conforms | Conforms | Conforms | Conforms |
| Assay, LACE | ATM-1405 | 90.0 - 110.0% of Label Claim | 101.1% | 107.1% | 106.7% | 98.9% | 98.1% | 95.0% | 94.7% |
| Related Compounds | ATM-1405 | Individual Report ≥ 0.05% (% area) Total Report | | | | | RRT 0.65:0.08% | | RRT 0.59:0.07% |
| | | | | | RRT 0.59:0.21% | | | RRT 0.61:0.34% | |
| | | | RRT 0.57:0.05% | RRT 0.58:0.17% | RRT 0.64:0.09% | RRT 0.61:0.10% | RRT 0.67:0.15% | RRT 0.67:0.21% | RRT 0.63:0.12% |
| | | | RRT 0.59:0.21% | RRT 0.63:0.09% | RRT 0.67:0.15% | RRT 0.66:0.07% | RRT 0.71:0.12% | RRT 0.69:0.23% | RRT 0.68:0.09% |
| | | | RRT 0.64:0.13% | RRT 0.66:0.17% | RRT 0.83:0.06% | RRT 0.86:0.07% | RRT 0.74:0.16% | RRT 0.84:0.06% | RRT 0.72:0.12% |
| | | | RRT 0.83:0.06% | RRT 0.83:0.06% | RRT 1.18:0.05% | RRT 1.21:0.08% | RRT 0.83:0.05% | RRT 1.14:0.08% | RRT 0.89:0.05% |
| | | | RRT 1.23:0.14% | RRT 1.23:0.16% | RRT 1.20:0.10% | RRT 1.27:0.09% | RRT 1.09:0.07% | RRT 1.20:0.10% | RRT 1.38:0.06% |
| | | | Total: 0.8% | LA: 0.06% | LA: 0.10% | LA: 0.17% | RRT 1.15:0.09% | LA: 0.32% | RRT 1.42:0.09% |
| | | | | Total: 0.7% | | Total: 0.6% | LA: 0.22% | | LA: 0.38% |
| | | | | | Total: 0.8% | | Total: 0.9% | Total: 1.3% | Total: 1.0% |
| Assay, preservative | ATM-1406 | Report | 0.0447 mg/mL | 0.0443 mg/mL | 0.0446 mg/mL | 0.0447 mg/mL | 0.0440 mg/mL | 0.0441 mg/mL | 0.0504 mg/mL |
| pH | USP <791> | Report | 4.6 | 4.5 | 4.5 | 4.5 | 4.5 | 4.4 | 4.3 |
| Osmolality | USP <785> | 250-350 mOsm/kg | 262 mOsm/kg | 263 mOsm/kg | 263 mOsm/kg | 262 mOsm/kg | 262 mOsm/kg | 261 mOsm/kg | 255 mOsm/kg |

| **25°C ± 5°** | | | | | | | | | |
|---|---|---|---|---|---|---|---|---|---|
| Appearance | ATM-1095 | Clear, pale yellow to yellow solution essentially free of foreign or particulate matter | Conforms | Conforms | Conforms | Conforms | Conforms | Conforms | |
| Assay, LACE | ATM-1405 | 90.0 - 110.0% of Label Claim | 101.1% | 107.3% | 107.0% | 97.2% | 98.9% | 87.1% | |
| Related Compounds | ATM-1405 | Individual Report ≥ 0.05% (% area) Total Report | | RRT 0.56:0.05% | | | RRT 0.66:0.28% | RRT 0.61:0.58% | |
| | | | RRT 0.57:0.05% | RRT 0.58:0.22% | RRT 0.59:0.27% | RRT 0.59:0.18% | RRT 0.72:0.15% | RRT 0.69:0.30% | |
| | | | | | RRT 0.64:0.12% | | RRT 0.74:0.21% | | |
| | | | RRT 0.59:0.21% | RRT 0.63:0.12% | | RRT 0.66:0.09% | | RRT 0.84:0.06% | |
| | | | RRT 0.64:0.13% | RRT 0.66:0.21% | RRT 0.67:0.18% | RRT 0.86:0.07% | RRT 0.83:0.06% | RRT 1.14:0.08% | |
| | | | | | RRT 0.83:0.07% | | RRT 1.09:0.07% | | |
| | | | RRT 0.67:0.21% | RRT 0.83:0.07% | RRT 1.20:0.16% | RRT 1.21:0.08% | RRT 1.15:0.20% | RRT 1.20:0.29% | |
| | | | RRT 0.83:0.06% | RRT 1.23:0.09% | | RRT 1.27:0.19% | | RRT 1.26:0.51% | |
| | | | RRT 1.23:0.14% | RRT 1.25:0.07% | RRT 1.35:0.09% | LA: 0.94% | RRT 1.21:0.27% | RRT 1.34:0.18% | |
| | | | Total: 0.8% | LA: 0.35% | LA:0.65% | Total: 1.6% | RRT 1.27:0.09% | LA: 1.18% | |
| | | | | Total: 1.2% | Total: 1.5% | | LA: 1.05% | Total: 3.2% | |
| | | | | | | | Total: 2.4% | | |
| Assay, preservative | ATM-1406 | Report | 0.0447 mg/mL | 0.0445 mg/mL | 0.0447 mg/mL | 0.0438 mg/mL | 0.0435 mg/mL | 0.0462 mg/mL | |
| pH | USP <791> | Report | 4.6 | 4.4 | 4.4 | 4.3 | 4.2 | 4.1 | |
| Osmolality | USP <785> | 250-350 mOsm/kg | 262 mOsm/kg | 264mOsm/kg | 264mOsm/kg | 263 mOsm/kg | 261 mOsm/kg | 259 mOsm/kg | |
| | | | | | | | | | |

| | | | | | | | | | |
|---|---|---|---|---|---|---|---|---|---|
| Related Compounds: LA = R-α-Lipoic Acid (USP Standard) | | | | | | | | | |

## Claims

1. A pharmaceutical composition for the treatment of presbyopia, comprising a pharmaceutically acceptable salt of 0.1-10% by weight of lipoic acid choline ester, 0.1-2% by weight of a tonicity adjusting agent, 0.003-0.01% by weight of a preservative, a buffer selected from the group consisting of acetate, borate and acetate/boric acid buffers, 0.05%-1.0% by weight of a biochemical energy source that is alanine, and water.

2. The composition of claim 1, wherein the pharmaceutically acceptable salt of lipoic acid choline ester is a chloride, bromide, or iodide salt.

3. The composition of claim 1 or 2, wherein the tonicity adjusting agent is glycerol.

4. The composition of any one of claims 1 to 3, wherein the composition is preservative free.

5. The composition of any one of claims 1 to 4, wherein the composition is free of benzalkonium chloride.

6. The composition of any one of claims 1 to 3, wherein the preservative is benzalkonium chloride.

7. The composition of any one of the claims 1 to 6, wherein the composition has a pH of 4 to 8.

8. The composition of any one claims 1 to 7, which has a shelf-stability of at least 3 months, at least 6 months, at least 9 months, or at least 1 year.

9. The composition of any one of claims 1 to 8, which is a non-irritating eye-drop solution.

10. A method of producing the pharmaceutical composition according to any one of claims 1-9, comprising:
finely grinding the pharmaceutically acceptable salt of lipoic acid choline ester into powder having an average particle size of no greater than 5 mm,
adding the ground salt to water, wherein the water has less than 5 ppm, preferably less than 2 ppm of oxygen,
mixing for 6 to 8 hours, preferably for 8 hours, at room temperature, to form a mixture,
filling an ophthalmic bottle with the mixture and adding an inert gas overlay before capping,
packaging the filled-and-capped ophthalmic bottle in a gas-impermeable pouch, and
storing the package at 2-8°C.

11. The method of claim 10, wherein the pouch contains an oxygen scavenger.

12. The method of any one of claims 10 to 11, wherein the mixing temperature is between 20 and 25°C.

13. The method of any one of claims 10 to 12, wherein the inert gas is nitrogen.

14. The method of any one of claims 10 to 13, wherein the ophthalmic bottle is a low density polypropylene (LDPE), polyethylene terephthalate (PET), or polytetrafluoroethylene (PTFE) bottle.

15. The method of any one of claims 10 to 14, wherein the ophthalmic bottle is a blow-fill-seal unit.

16. A shelf stable and/or non-irritating eye-drop solution prepared by any one of the method of claims 10 to 15.

## Patentansprüche

1. Pharmazeutische Zusammensetzung zur Behandlung von Presbyopie, umfassend ein pharmazeutisch unbedenkliches Salz von 0,1-10 Gew.-% Liponsäurecholinester, 0,1-2 Gew.-% eines Mittels zum Einstellen der Tonizität, 0,003-0,01 Gew.-% eines Konservierungsstoffs, einen aus der aus Acetat-, Borat- und Acetat/Borsäure-Puffern bestehenden Gruppe ausgewählten Puffer, 0,05 Gew.-%-1,0 Gew.-% einer biochemischen Energiequelle, bei der es sich um Alanin handelt, und Wasser.

2. Zusammensetzung nach Anspruch 1, wobei es sich bei dem pharmazeutisch unbedenklichen Salz von Liponsäurecholinester um ein Chlorid-, Bromid- oder Iodidsalz handelt.

3. Zusammensetzung nach Anspruch 1 oder 2, wobei es sich bei dem Mittel zum Einstellen der Tonizität um Glycerin handelt.

4. Zusammensetzung nach einem der Ansprüche 1 bis 3, wobei die Zusammensetzung konservierungsstofffrei ist.

5. Zusammensetzung nach einem der Ansprüche 1 bis 4, wobei die Zusammensetzung frei von Benzalkoniumchlorid ist.

6. Zusammensetzung nach einem der Ansprüche 1 bis 3, wobei es sich bei dem Konservierungsstoff um Benzalkoniumchlorid handelt.

7. Zusammensetzung nach einem der Ansprüche 1 bis 6, wobei die Zusammensetzung einen pH-Wert von 4 bis 8 aufweist.

8. Zusammensetzung nach einem der Ansprüche 1 bis 7 mit einer Haltbarkeit von mindestens 3 Monaten, mindestens 6 Monaten, mindestens 9 Monaten oder mindestens 1 Jahr.

9. Zusammensetzung nach einem der Ansprüche 1 bis 8, bei der es sich um eine nichtreizende Augentropfenlösung handelt.

10. Verfahren zum Herstellen der pharmazeutischen Zusammensetzung nach einem der Ansprüche 1-9, bei dem man:
das pharmazeutisch unbedenkliche Salz von Liponsäurecholinester fein zu einem Pulver mit einer durchschnittlichen Teilchengröße von nicht mehr als 5 mm zermahlt,
das gemahlene Salz zu Wasser gibt, wobei das Wasser weniger als 5 ppm, vorzugsweise weniger als 2 ppm, Sauerstoff aufweist,
6 bis 8 Stunden, vorzugsweise 8 Stunden, bei Raumtemperatur mischt, unter Bildung einer Mischung,
die Mischung in eine ophthalmische Flasche füllt und vor dem Verschließen eine Deckschicht aus inertem Gas zufügt,
die gefüllte und verschlossene ophthalmische Flasche in einem gasundurchlässigen Beutel verpackt und
die Packung bei 2-8°C lagert.

11. Verfahren nach Anspruch 10, wobei der Beutel einen Sauerstofffänger enthält.

12. Verfahren nach Anspruch 10 oder 11, wobei die Mischtemperatur zwischen 20 und 25°C liegt.

13. Verfahren nach einem der Ansprüche 10 bis 12, wobei es sich bei dem inerten Gas um Stickstoff handelt.

14. Verfahren nach einem der Ansprüche 10 bis 13, wobei es sich bei der ophthalmischen Flasche um eine Flasche aus Polypropylen niedriger Dichte (Low Density Polypropylene, LDPE), Polyethylenterephthalat (PET) oder Polytetrafluorethylen (PTFE) handelt.

15. Verfahren nach einem der Ansprüche 10 bis 14, wobei es sich bei der ophthalmischen Flasche um eine Blow-Fill-Seal-Einheit handelt.

16. Haltbare und/oder nichtreizende Augentropfenlösung, hergestellt durch das Verfahren nach einem der Ansprüche 10 bis 15.

## Revendications

1. Composition pharmaceutique pour le traitement de la presbytie comprenant un sel pharmaceutiquement acceptable de 0,1 à 10 % en poids d'ester de choline d'acide lipoïque, 0,1 à 2 % en poids d'un agent d'ajustement de la tonicité, 0,003 à 0,01 % en poids d'un conservateur, un tampon choisi dans le groupe constitué par des tampons acétate, borate et acétate/acide borique, 0,05 % à 1,0 % en poids d'une source d'énergie biochimique qui est l'alanine, et de l'eau.

2. Composition pharmaceutique selon la revendication 1, le sel pharmaceutiquement acceptable d'ester de choline d'acide lipoïque étant un sel de chlorure, de bromure, ou d'iodure.

3. Composition selon la revendication 1 ou 2, l'agent d'ajustement de la tonicité étant le glycérol.

4. Composition selon l'une quelconque des revendications 1 à 3, la composition étant exempte de conservateur.

5. Composition selon l'une quelconque des revendications 1 à 4, la composition étant exempte de chlorure de benzalkonium.

6. Composition selon l'une quelconque des revendications 1 à 3, le conservateur étant le chlorure de benzalkonium.

7. Composition selon l'une quelconque des revendications 1 à 6, la composition possédant un pH de 4 à 8.

8. Composition selon l'une quelconque des revendications 1 à 7, qui possède une stabilité au stockage d'au moins 3 mois, d'au moins 6 mois, d'au moins 9 mois, ou d'au moins 1 an.

9. Composition selon l'une quelconque des revendications 1 à 8, qui est une solution non irritante de gouttes oculaires.

10. Procédé de production de la composition pharmaceutique selon l'une quelconque des revendications 1 à 9, comprenant :
le broyage fin du sel pharmaceutiquement acceptable d'ester de choline d'acide lipoïque en une poudre possédant une taille moyenne de particule non supérieure à 5 mm,
l'ajout du sel broyé à de l'eau, l'eau possédant moins de 5 ppm, préférablement moins de 2 ppm d'oxygène,
le mélange pendant 6 à 8 heures, préférablement pendant 8 heures, à température ambiante, pour former un mélange,
le remplissage d'une bouteille ophtalmique avec le mélange et l'ajout d'un recouvrement de gaz inerte avant capsulage,
l'emballage de la bouteille ophtalmique remplie et capsulée dans une poche imperméable aux gaz, et
le stockage de l'emballage à une température de 2 à 8 °C.

11. Procédé selon la revendication 10, la poche contenant un capteur d'oxygène.

12. Procédé selon l'une quelconque des revendications 10 et 11, la température de mélange étant comprise entre 20 et 25 °C.

13. Procédé selon l'une quelconque des revendications 10 à 12, le gaz inerte étant l'azote.

14. Procédé selon l'une quelconque des revendications 10 à 13, la bouteille ophtalmique étant une bouteille de polypropylène basse densité (LDPE), de poly(téréphtalate d'éthylène) (PET), ou de polytétrafluoroéthylène (PTFE).

15. Procédé selon l'une quelconque des revendications 10 à 14, la bouteille ophtalmique étant une unité de type soufflage-remplissage-scellage.

16. Solution de gouttes oculaires stable au stockage et/ou non irritante préparée par l'un quelconque des procédés selon les revendications 10 à 15.
